# EUROPÄISCHE PATENTSCHRIFT

(11) **EP 4 114 818 B1**
(45) Veröffentlichungstag und Bekanntmachung des Hinweises auf die Patenterteilung: **31.05.2023**
(21) Anmeldenummer: 21789672.9
(22) Anmeldetag: 07.10.2021
(51) Int. Cl.: C07C 51/06, C07C 57/04, C07C 67/20, C07C 69/54, C07C 231/06, C07C 231/12, C07C 233/09, C07C 303/24, C07C 305/06

(54) **VERBESSERTES VERFAHREN ZUR HERSTELLUNG VON METHYLMETHACRYLAT UND/ODER METHACRYLSÄURE DURCH REDUZIERTE RÜCKVERMISCHUNG IN DER KONVERTIERUNG**
IMPROVED METHOD FOR THE PREPARATION OF METHYL METHACRYLATE AND / OR METHACRYLIC ACID BY REDUCED BACK-MIXING DURING CONVERSION
PROCÉDÉ AMÉLIORÉ DE FABRICATION DE MÉTHACRYLATE DE MÉTHYLE ET/OU D'ACIDE MÉTHACRYLIQUE PAR RÉDUCTION DU MÉLANGE EN CONVERSION

(30) Priorität: 23.10.2020 EP 20203722
(43) Veröffentlichungstag der Anmeldung: 11.01.2023
(73) Patentinhaber: Röhm GmbH, 64295 Darmstadt (DE)
(72) Erfinder: KOCKESEN, Ufuk, 53859 Niederkassel (DE); WINGS, Patrick, 50935 Köln (DE); KRILL, Steffen, 64367 Mühltal (DE); KLASOVSKY, Florian, 45721 Haltern am See (DE)
(74) Vertreter: Röhm Patent Association
(86) Internationale Anmeldenummer: PCT/EP2021/077640
(87) Internationale Veröffentlichungsnummer: WO 2022/084046

(56) Entgegenhaltungen:
- WO-A1-99/41228
- WO-A1-2015/055844
- DE-A1-102004 006 826

## Beschreibung

Die vorliegende Erfindung betrifft ein Verfahren zur Herstellung von Methylmethacrylat (MMA) und/oder Methacrylsäure (MAS) mit verbesserter Ausbeute umfassend die Verfahrensschritte Amidierung, Konvertierung und Hydrolyse/Veresterung, wobei insbesondere hohe Ausbeuten im Schritt der Amidierung (auch als Hydrolyse von Acetoncyanhydrin bezeichnet) und im nachfolgenden Verfahrensschritt der sogenannten Konvertierung erzielt werden. Die Konvertierung umfasst die Umsetzung der in der Amidierung erhaltenen schwefelsauren Reaktionsmischungen, welche einen hohen Anteil von α-Sulfoxyisobuttersäureamid (SIBA) enthält, zu einer schwefelsauren, Methacrylsäureamid (MASA) enthaltenden Reaktionsmischung. Durch die erfindungsgemäße konstruktive Ausführung der im Konvertierungsschritt verwendeten Reaktoren, insbesondere Wärmeumwandlungsapparaturen, in Verbindung mit einer optimierten Konzentration der eingesetzten Schwefelsäure ist ein deutlicher Ausbeutegewinn an MASA gegenüber dem Stand der Technik möglich. Weiterhin kann eine Optimierung mit Hilfe einer Kühlung der Rektionsmischung erzielt werden, die zwischen Konvertierung und Veresterung erfolgt. Die optimierten MASA-Ausbeuten sind zudem deutlich geringeren Schwankungen unterworfen und resultieren in einer erhöhten Gesamtausbeute zu MMA bzw. MAS. Die Ausbeuten können zudem auch bei Teillastbetrieb die mit anderen Verfahren erreichbaren Ausbeuten übertreffen.

Die Erfindung umfasst spezielle strömungs-optimierte apparative Konstruktionen im Konvertierungsschritt in Kombination mit der optimalen Einstellung verfahrenstechnischer, stöchiometrischer Parameter, insbesondere die Kontrolle und Optimierung der Schwefelsäurekonzentration.

### Stand der Technik

Es sind mehrere kommerzielle Verfahren bekannt, um im industriellen Maßstab Alkylmethacrylate, insbesondere MMA, herzustellen. Ein weltweit verbreitetes, kommerzielles Verfahren basiert auf Aceton als Grundstoff, und wird meist als C3-Prozess oder ACH-Sulfo-Prozess bezeichnet. Hierbei wird Aceton mit Blausäure (HCN) zum zentralen Zwischenprodukt Acetoncyanhydrin (ACH) umgesetzt. Dieses Zwischenprodukt wird isoliert und für die folgenden Prozessschritte zur Herstellung von MAS und MMA eingesetzt.

Ein typischer Prozess zur Herstellung von MMA ausgehend von ACH ist beispielsweise in US 4,529,816 beschrieben. WO 2015/055844 A1 offenbart die Reaktion von Acetoncyanhydrin mit Schwefelsäure zur Herstellung einer Mischung, die Sulfoxyisobuttersäureamid enthält, das thermisch in Methacrylsäureamid umgewandelt wird. Methacrylsäureamid wird mit Methanol oder Wasser reagiert, um Methylmethacrylat oder Methacrylsäure herzustellen. In einer Vielzahl weiterer Dokumente wird beschrieben, wie die verschiedenen Aspekte der Reaktionsteilschritte verbessert und optimiert werden können, wobei letztlich die Gesamtausbeute an den gewünschten Monomeren MAS und MMA erhöht werden soll.

Beim ACH-Sulfo-Prozess wird ACH mit Hilfe von Schwefelsäure unter Bildung von α-Hydroxyisobuttersäureamid (HIBA) und seines Sulfatesters (SIBA) hydrolysiert. Dieser Teilschritt wird in der einschlägigen Literatur als Amidierung oder auch Hydrolyse bezeichnet, da die Nitril-Gruppe des ACH formal in eine Amid-Funktion umgewandelt wird.

HIBA, SIBA und bereits in der Amidierung gebildetes MASA werden anschließend thermisch in MASA und kleinere Mengen an MAS in der schwefelsauren Reaktionsmischung umgewandelt. Dieser Teilschritt wird in der einschlägigen Literatur als Konvertierung bezeichnet, wobei die Sulfoxygruppe des SIBA im Sinne einer ß-Eliminierung formal in eine olefinische Doppelbindungsfunktion umgewandelt wird, wobei MASA aus SIBA hervorgeht und formal freie Schwefelsäure wieder freigesetzt wird.

Die nach Konvertierung erhaltene schwefelsaure MASA-Lösung kann dann mit Methanol und Wasser versetzt werden, um durch eine Veresterungsreaktion MMA herzustellen. Alternativ hierzu kann die nach der Konvertierung erhaltene schwefelsaure MASA-Lösung mit Wasser zur Methacrylsäure (MAS) umgesetzt werden (Hydrolyse). Restliches HIBA wird hierbei typischerweise teilweise zu Hydroxyisobuttersäuremethylester (HIBSM) umgesetzt, zum Teil aber auch zu Hydroxyisobuttersäure (HIBS) hydrolysiert.

In der Konvertierung erfolgt die Umwandlung von SIBA zu MASA leichter als die Umwandlung von HIBA zu MASA. Um die thermische Umwandlung von HIBA zu MASA zu beschleunigen, müssten sowohl Wärme als auch eine längere Verweilzeit bereitgestellt werden. Allerdings ist dieser Prozess wiederum mit einem Abbau von ausbeuterelevanten Zwischenprodukten wie MASA verbunden, wobei sich teerartige Ablagerungen bilden können sowie eine Vielzahl konsekutiv entstehender Verbindungen, die zu einer Verminderung der Ausbeute beitragen. Die Reaktionsbedingungen müssen daher dahingehend optimiert werden, so dass insbesondere die gewünschte Hauptreaktion der Konvertierung, nämlich die Umwandlung SIBA → MASA, sowie die ebenfalls gewünschte Umwandlung des Nebenprodukts HIBA zu MASA gefördert werden. Eine Abnahme der thermischen Umwandlung in gewünschte Produkte führt typischerweise zu einer verringerten Gesamtausbeute für das Verfahren.

Das Verfahren zur Herstellung von MAS hat im Wesentlichen ähnliche Merkmale wie das zur Herstellung von MMA. Bei der MAS-Herstellung und der MMA-Herstellung sind oftmals Amidierung und Konvertierung grundsätzlich ähnlich oder identisch, wobei in beiden Fällen eine möglichst hohe MASA-Ausbeute aus ACH gewünscht ist. Wenn die MASA-haltige Reaktionsmischung ohne Alkohol nur mit Wasser umgesetzt wird, führt die Reaktion (Hydrolyse von MASA) typischerweise zu MAS-Rohgemischen. Dagegen führt die Umsetzung (Veresterung) der MASA-haltige Reaktionsmischung in Gegenwart von Wasser und Alkohol (z.B. Methanol) zu Alkylmethacrylaten (z.B. MMA). Dem Fachmann ist in diesem Zusammenhang bekannt, dass für die selektive Herstellung von MAS höhere Stoffmengenanteile an Wasser zur Verfügung gestellt werden müssen, als dies bei der MMA-Herstellung der Fall ist.

Sowohl die MAS-Herstellung als auch die Produktion von MMA werden in Anlagenkomplexen betrieben, die oftmals bis zu mehrere 100.000 Tonnen an Produkt pro Jahr herstellen. Insofern können bereits geringfügige Verbesserungen der Ausbeute zu signifikanten Einsparungen führen. Ein nicht zu unterschätzender Nebeneffekt für den ökologischen Footprint der Verfahren liegt zudem in der Betrachtung der Abfallstoffe bei diesen Verfahren. In der Regel werden erhebliche Mengen an Abfallsäure erzeugt, die oftmals mit hohem energetischem Aufwand wieder regeneriert werden müssen, um die Schwefelsäure als Ausgangsstoff zurückzugewinnen. Teerartige Verunreinigungen in der Abfallsäure müssen in der Regel von Zeit zu Zeit teilweise manuell entfernt und aufwändig entsorgt werden, was insbesondere hohe Ansprüche an Technik und Arbeitssicherheit stellt.

Die Problematik der Verweilzeitkontrolle in rohrartigen Reaktoren bei hohen Temperaturen, die Stoffumwandlungen erlauben, ist im Stand der Technik in einer Vielzahl von Patenten und Publikationen beschrieben. So offenbart beispielsweise die EP 0 202 099 A ein Verfahren zum Behandeln von Schwerölrückständen. Hierbei werden Schwerölrückstände einem thermischen Cracken unterzogen, wobei ein aus Rohren bestehender thermischer Reaktor verwendet wird, der ein pfropfenförmiges Strömungsprofil aufweist. Resultierende Crackprodukte werden mittels Strippung entfernt.

US 5,393,918 beschreibt einen Ansatz, die Ausbeute eines MMA-Herstellungsverfahrens zu verbessern. Hierbei wird allerdings nicht der Konvertierungsschritt bzw. die MASA-Ausbeute verbessert, sondern die Umwandlung eines Nebenprodukts der Veresterung, nämlich des Hydroxyisobuttersäuremethylesters (HIBSM), zu zusätzlichen Mengen an MMA durchgeführt. US 5,393,918 beschreibt ein Verfahren, bei dem HIBA und SIBA in dem auf die Amidierung folgenden Veresterungsschritt zu den beiden Konfigurationsisomeren des Methoxyisobuttersäuremethylesters (α-MIBSM bzw. β-MIBSM) umgewandelt werden. Diese drei Nebenprodukte werden in der MMA-Aufarbeitung weitestgehend von MMA abgetrennt und in einem separaten Dehydratisierungsschritt in MMA umgewandelt. Diese aufwändige Verfahrensführung eliminiert die Notwendigkeit einer thermischen Umwandlung (Konvertierung) von HIBA und SIBA in MASA, erfordert jedoch eine fraktionierte Destillation sowie einen anschließenden zusätzlichen Dehydratisierungsschritt. Das Verfahren nach US 5,393,918 ist somit aus technischer Sicht komplex, aufwändig und erfordert zusätzliche Apparate und Reaktoren.

Die thermische Umwandlung von HIBA und SIBA zu MASA (Konvertierung) wird typischerweise in rohrförmigen Reaktoren durchgeführt, die im Stand der Technik teilweise als Crackreaktoren bezeichnet werden. Diese Bezeichnung verdeutlicht die thermisch anspruchsvollen Bedingungen und die gewünschte β-Eliminierungsreaktion. Diese Reaktoren zur Durchführung der Konvertierung enthalten in der Regel als apparative Elemente mindestens einen Wärmetauscher zur Bereitstellung der für die Reaktion erforderlichen Energie sowie Verweilzeit-Apparate, die die erforderliche Verweilzeit für die MASA-Bildung unter den erforderlichen Bedingungen bereitstellen.

Eine typische thermische Umwandlungsvorrichtung und apparative Ausführungsform eines Konvertierungsreaktors sind beispielsweise in EP 0 999 200 beschrieben. Der hier beschriebene Konvertierungsreaktor umfasst ein Metallrohr mit mehreren Durchführungen und Öffnungen. In einer Ausführungsform kann das Metallrohr eine Scheidewand enthalten, welche das Rohr trennt, um einen Durchgang mit einem Bogenrohr-Element (180 °-Umleitung des Reaktionsgemischs) bereitzustellen. Oftmals dient die Ausgestaltung des Rohrreaktors mit Umlenkungen dazu, den Platzbedarf des Konvertierungsreaktors zu minimieren. Der Reaktor, in dem die Umsetzung der SIBA haltigen Amidierungsmischung zu MASA durchgeführt wird, kann beispielsweise zusammengesetzt sein aus rohrförmigen, begleitbeheizten Elementen sowie umlenkenden Rohrelementen, welche gegebenenfalls mit Flanschen miteinander verbunden sind. Der hier beschriebene Konvertierungsreaktor weist zudem eine Erweiterung an der Stelle auf, an der die Amidierungsmischung in die thermische Umwandlungsvorrichtung eintritt, und weist eine Verengung auf an der Stelle, an der die Konvertierungsmischung die thermische Umwandlungsvorrichtung verlässt.

Im Stand der Technik, wie z.B. in EP 0 999 200, sind verschiedene Probleme zu erkennen. Gemäß EP 0 999 200 werden Ausbeuten nach dem Amidierungsschritt von 96 % bis 97 % sowie Ausbeuten nach dem Konvertierungsschritt in der Größenordnung von 92 % bis 94 % erreicht. Auffallend sind insbesondere Ausbeuteschwankungen um bis zu 2 %, so dass im technischen Maßstab mit den beschriebenen Maßnahmen kein ausreichend stabiler Prozessbetrieb aufrechterhalten werden kann. Die in EP 0 999 200 beschriebenen Ausbeuten werden erreicht durch die spezielle Durchführung der Konvertierung, unter Gewährleistung einer Plug-Flow-Strömung und einer basischen Waschmethode des Veresterungsgemischs. Grundsätzlich erfordert die Ausgestaltung in EP 0 999 200 einen hohen apparativen Aufwand.

Diese konstruktiven Merkmale einer typischen thermischen Umwandlungsvorrichtung führen zu einer Rückvermischung von HIBA, SIBA, MASA und MAS, die in der Amidierungsmischung aus ACH vorgebildet sind. Die Rückvermischung dieser Zwischenprodukte resultiert unter anderem aus Geschwindigkeitsänderungen an den Rohrübergängen. Insbesondere führt eine Störung der propfenförmigen Strömung zu einer verringerten Gesamtausbeute, da die Verweilzeit der Komponenten im Konvertierungsreaktor variiert. Eine propfenförmige Strömung ist, wie dem Fachmann bekannt ist, durch parabolisches Geschwindigkeitsprofil gekennzeichnet, bei dem die Moleküle an der Rohrinnenwand eine geringere Geschwindigkeit aufweisen, als Moleküle im Kern der Strömung bzw. in der Rohrmitte. Mit steigendem Durchfluss durch die Rohrleitung (und steigender Reynolds-Zahl) flacht die Parabel der Geschwindigkeitsverteilung über den Rohrquerschnitt ab. Im Stand der Technik ist der Zusammenhang zwischen Strömungsregime (z.B. Einfluss der Reynolds-Zahl) und dem Reaktionsverlauf der Amidierung nicht ausreichend beschrieben.

Unzureichende und/oder schwankende Ausbeuten im Konvertierungsreaktor haben oftmals folgende Ursache: Statistisch weisen bestimmte Anteile der in der Reaktionsmischung vorhandenen Komponenten eine Verweilzeit im Konvertierungsreaktor auf, die deutlich größer bzw. deutlich geringer ist als die für die vollständige Durchführung der Zielreaktionen notwendige Verweilzeit. Während zu geringe Verweilzeiten oftmals dazu führt, dass es zu einer nicht vollständigen Umsetzung von SIBA bzw. HIBA zu MASA kommt, führen hohe Verweilzeiten oftmals dazu, dass die oben beschriebenen thermischen Abbauprodukte gebildet werden. Beide Ausprägungen einer unzureichenden Kontrolle der Verweilzeit im Konvertierungsreaktor tragen somit typischerweise zu einer Erniedrigung der erzielbaren Ausbeute an Zielprodukt bei.

Die vorteilhafte Verwendung eines Rohrreaktors zur Bereitstellung einer pfropfenförmigen Strömung bei der Herstellung von Methacrylsäureestern wird in US 4,748,268 A beschrieben. Hier wird beschrieben, wie ein Stoffstrom, welcher Methacrylsäure, einen C₁-C₄-Alkohol, einen Katalysator und eine flüssige organische Substanz enthält, in eine Pfropfenströmungsapparatur eingespeist wird. Diese Beschreibung umfasst jedoch lediglich die Veresterung von Methacrylsäure, und nicht die Bildung von Methacrylsäureestern aus HIBA oder SIBA umfassend einen Konvertierungsschritt.

Somit besteht weiterhin Bedarf an einem kommerziellen Verfahren mit verbesserter Ausbeute zur Herstellung von MMA oder MAA, insbesondere in den Teilschritten Amidierung (Hydrolyse) und Konvertierung.

### Aufgabe

Der vorliegenden Erfindung lag die Aufgabe zugrunde, mit geringem Investitionsaufwand und einem gleichbleibend hohen Maß an Betriebssicherheit die Ausbeute des ACH-Sulfo-Verfahrens zur Herstellung von Methacrylamid sowie dessen Folgeprodukten Methylmethacrylat und Methacrylsäure nachhaltig zu steigern. Insbesondere war es Aufgabe der vorliegenden Erfindung, Phänomene der Rückvermischung in den thermischen Umwandlungsschritten der MMA-Herstellung zu minimieren und die Bildung von Nebenprodukten und somit von Abfall zu reduzieren.

### Lösung

Die Aufgabe wurde dadurch gelöst, dass im erfindungsgemäßen Verfahren durch die Minimierung der Rückvermischung und durch die gezielte Kontrolle der Verweilzeit in der Konvertierung die thermische Umwandlung von HIBA und SIBA zu MASA signifikant verbessert und daher eine erhöhte Gesamtprozessausbeute des Herstellverfahrens erhalten wird. Insbesondere wird die Rückvermischung durch das möglichst weitgehende Aufrechterhalten einer so genannten Pfropfenströmung vermindert, wobei mit Pfropfenströmung gemeint ist, dass die Geschwindigkeit des Fluids, z.B. der Reaktionsmischung, im Rohr über den gesamten Rohrquerschnitt nahezu konstant ist. Es wurde gefunden, dass durch eine neuartige Kombination von apparativen Vorrichtungen zur Strömungsoptimierung und verfahrenstechnischen Parametern, wie insbesondere der Konzentration der eingesetzten Schwefelsäure, eine optimierte Ausbeute bei der Konvertierung und damit auch beim gesamten Verfahren erhalten werden kann.

Es wurde zudem überraschenderweise gefunden, dass bei zu starker thermischer Belastung und zu langen Verweilzeiten nicht unerhebliche Mengen an MASA-Dimeren und -Oligomeren gebildet werden, sowie auch Misch-Dimere und -Oligomere aus MASA und MAS. Zur Erzielung einer hohen MMA-Ausbeute ist weiterhin die Minimierung der Konzentration an freier MAS in der Konvertierungsmischung wünschenswert, da diese unter den thermischen Bedingungen der Konvertierung ebenfalls zu unerwünschten Folgereaktionen neigt.

Durch fluiddynamische Simulation von Strömung und Vermischung konnten die Schwachstellen des Konvertierung-Verfahrensschritts und insbesondere die Abweichungen von gewünschten Parametern (z.B. der Verweilzeit) von den festgelegten idealen Bedingungen sichtbar gemacht werden. Durch die erfindungsgemäßen konstruktiven Anpassungen des Konvertierungsreaktors, insbesondere der thermischen Umwandlungsapparatur, konnten diese Schwachstellen behoben werden. Insbesondere gelingt es, durch gezielte Kombination einer verminderten Rückvermischung und eines gleichverteilen Strömungsprofils im Konvertierungsschritt des Verfahrens die thermische Umwandlung von SIBA und HIBA zu MASA signifikant zu verbessern und damit die gewünschte Ausbeutesteigerung an MMA oder MAS zu erzielen.

Die Erfindung und ihre kennzeichnenden Merkmale sind im Folgenden eingehend beschrieben.

### Beschreibung der Erfindung

Die vorliegende Erfindung betrifft ein Verfahren zur Herstellung von Methylmethacrylat und/oder Methacrylsäure, umfassend die Verfahrensschritte:
a. die Umsetzung von Acetoncyanhydrin und Schwefelsäure in ein oder mehreren Reaktoren I in einer ersten Reaktionsstufe (Amidierung) bei einer Temperatur im Bereich von 70 °C bis 130°C, wobei eine erste Reaktionsmischung, enthaltend Sulfoxyisobuttersäureamid (SIBA) und Methacrylsäureamid (MASA), erhalten wird;
b. das Konvertieren der ersten Reaktionsmischung, umfassend das Erhitzen auf eine Temperatur im Bereich von 130 bis 200 °C, in ein oder mehreren Reaktoren II in einer zweiten Reaktionsstufe (Konvertierung), wobei eine zweite Reaktionsmischung, enthaltend überwiegend Methacrylsäureamid und Schwefelsäure, erhalten wird; und
c. die Umsetzung der zweiten Reaktionsmischung mit Wasser und optional Methanol, in ein oder mehreren Reaktoren III in einer dritten Reaktionsstufe (Veresterung oder Hydrolyse), wobei eine dritte Reaktionsmischung, enthaltend Methacrylsäure und/oder Methylmethacrylat, erhalten wird;
wobei
(i) die in der ersten Reaktionsstufe eingesetzte Schwefelsäure, welche an einer oder mehreren Stellen der Reaktoren I zugeführt wird, eine Konzentration im Bereich von 98,0 Gew.-% bis 100,5 Gew.-% aufweist,
(ii) die Verweilzeit der ersten Reaktionsmischung in der zweiten Reaktionsstufe im Bereich von 2 bis 15 min liegt,
(iii) das Erhitzen in der zweiten Reaktionsstufe in einem oder mehreren Reaktoren II durchgeführt wird, wobei mindestens ein Reaktor II mindestens ein Vorheizer-Segment umfasst, welches ein oder mehrere lineare Rohrleitungselemente umfasst, die mittels eines räumlich von der Reaktionsmischung getrennten Heizmediums aufgeheizt werden, wobei die Reaktionsmischung um 10 bis 100°C erhitzt wird,
(iv) das Konvertieren in der zweiten Reaktionsstufe in einem oder mehreren Reaktoren II durchgeführt wird, wobei mindestens ein Reaktor II mindestens ein Verweilzeit-Segment umfasst, welches annähernd adiabatisch betrieben wird,
(v) das Vorheizer-Segment und/oder das Verweilzeit-Segment in einer Kombination aus linearen Rohrleitungselementen und Umlenkungen realisiert sind, wobei die linearen Rohrleitungselementen und die Umlenkungen über Reduzierflansche miteinander verbunden sind,
(vi) die in Schritt b erhaltene zweite Reaktionsmischung, enthaltend überwiegend Methacrylsäureamid und Schwefelsäure, optional auf eine Temperatur unterhalb von 120 °C abgekühlt wird, beispielsweise in einem Kühler mit einem Kühlmedium mit einer Temperatur im Bereich von 60 bis 100°C, und/oder optional in einem Zwischenbehälter zwischengepuffert wird, bevor die Reaktionsmischung in die dritte Reaktionsstufe geführt wird,
wobei das Leervolumen der linearen Rohrleitungselemente relativ zum Leervolumen der Umlenkungen in mindestens einem Vorheizer-Segment einem Verhältnis im Bereich von 2,0 bis 75,0 entspricht.

Insbesondere betrifft die vorliegende Erfindung ein optimiertes Verfahren zur Herstellung von Methylmethacrylat und/oder Methacrylsäure, umfassend die spezifische Einstellung und Kontrolle der Verweilzeit in den Schritten der Amidierung und/oder Konvertierung, insbesondere der Konvertierung, wobei einerseits die unzureichende Umsetzung ausbeuterelevanter Zwischenprodukte wie SIBA und HIBA durch eine zu geringe Verweilzeit und andererseits die ausbeutevermindernde Zersetzung reaktiver Zwischenprodukte durch eine zu hohe Verweilzeit in den genannten Verfahrensschritten unterbunden und gleichzeitig die Ausbeute an den Produkten MASA sowie MAS und damit letztendlich auch an MMA verbessert wird.

Im Sinne der vorliegenden Erfindung meint der Ausdruck "ppm", ohne weitere Angaben, Gew.-ppm (z.B. mg/kg).

Der Ausdruck Strom, Phase oder Fraktion enthaltend ein Edukt, Produkt und/oder Nebenprodukt ist im Sinne der Erfindung so zu verstehen, dass die genannte(n) Verbindung(en) in dem jeweiligen Strom enthalten ist (sind), beispielsweise ist der überwiegende Anteil des Edukts, Produkts und/oder Nebenprodukts in dem entsprechenden Strom zu finden. Grundsätzlich können neben den genannten Verbindungen weitere Bestandteile enthalten sein. Oftmals dient die Nennung der Bestandteile der Verdeutlichung des jeweiligen Verfahrensschritts.

Der Ausdruck "Brüden" oder "Brüdenstrom" bezeichnet im Sinne der Erfindung einen gasförmigen Verfahrensstrom, beispielsweise einen gasförmigen Kopfstrom einer Destillationskolonne.

Im Sinne der Erfindung meint der Ausdruck "adiabatisch" oder "adiabat", dass kein oder vernachlässigbarer Wärmeaustausch mit der Umgebung stattfindet.

### Erste Reaktionsstufe (Amidierung)

Das erfindungsgemäße Verfahren umfasst als Schritt (a) die Umsetzung von Acetoncyanhydrin (ACH) und Schwefelsäure in ein oder mehreren Reaktoren I in einer ersten Reaktionsstufe (Amidierung) bei einer Temperatur im Bereich von 70 bis 130°C, bevorzugt 70 bis 120 °C, wobei eine erste Reaktionsmischung, enthaltend Sulfoxyisobuttersäureamid und Methacrylsäureamid, erhalten wird.

Erfindungsgemäß weist die in der ersten Reaktionsstufe eingesetzte Schwefelsäure eine Konzentration im Bereich von 98,0 Gew.-% bis 100,5 Gew.-%, bevorzugt 98,0 Gew.-% bis 100,0 Gew.-%, bevorzugt von 99,0 Gew.-% bis 99,9 Gew.-%, bevorzugt von 99,3 bis 99,9 Gew.-%, insbesondere bevorzugt von 99,3 bis 99,8 Gew.-% auf. Insbesondere bezieht sich die angegebene Konzentration der eingesetzten Schwefelsäure auf die Gesamtmasse des Schwefelsäure-Zuleitungsstroms zur ersten Reaktionsstufe (z.B. (2)). Der Einsatz einer Schwefelsäure ohne Anteil an freiem SO₃, insbesondere einer Schwefelsäure mit einem Wasseranteil von 0,2 bis 0,7 Gew.-% hat sich als besonders vorteilhaft erwiesen.

Dem Fachmann sind grundsätzlich Methoden zur Bestimmung des Wassergehaltes von Stoffströmen, beispielsweise von Schwefelsäure-Zuleitungsströmen, bekannt. Beispielsweise kann der Wassergehalt von Stoffströmen durch Massenbilanzen, durch Messung der Dichte oder Schallgeschwindigkeit, gaschromatographisch, durch Karl-Fischer-Titration oder durch mittels HPLC ermittelt werden.

Das eingesetzte ACH kann mittels bekannter technischer Verfahren hergestellt werden (siehe beispielsweise Ullmanns Enzyklopädie der technischen Chemie, 4. Auflage, Band 7). Typischerweise werden Blausäure und Aceton in Gegenwart eines basischen Katalysators, z.B. eines Amins, in einer exothermen Reaktion zu ACH umgesetzt. Eine solche Verfahrensstufe ist beispielsweise in DE 10 2006 058 250 und DE 10 2006 059 511 beschrieben.

Typischerweise entstehen bei der Amidierung von ACH und Schwefelsäure als Hauptprodukte α-Hydroxyisobuttersäureamid (HIBA) bzw. dessen Hydrogensulfat (HIBA·H₂SO₄), Schwefelsäureester von HIBA (α-Sulfoxyisobuttersäureamid, SIBA) bzw. dessen Hydrogensulfat (SIBA·H₂SO₄) und Methacrylsäureamid-Hydrogensulfat (MASA·H₂SO₄) als Lösung in überschüssiger Schwefelsäure. Es ist dem Fachmann bekannt, dass die Anteile der genannten Komponenten in der Reaktionsmischung variabel sind und von den Reaktionsbedingungen abhängen.

Bevorzugt wird die erste Reaktionsstufe mit einem Überschuss an Schwefelsäure betreiben. Die Schwefelsäure dient bevorzugt als Lösemittel. Gleichzeitig dient die Schwefelsäure als Reaktant (für die Formierung des Zwischenprodukts SIBA) und als Katalysator der Umsetzung. Der Schwefelsäure-Überschuss kann insbesondere dazu dienen, die Viskosität der Reaktionsmischung niedrig zu halten, wodurch eine schnellere Abfuhr von Reaktionswärme und eine niedrigere Temperatur der Reaktionsmischung gewährleistet werden können. Dies kann insbesondere deutliche Ausbeutevorteile mit sich bringen. Obwohl Viskosität und Lösevermögen mit mehr Schwefelsäure verbessert werden, was letztlich auch eine allgemeine Selektivitätserhöhung mit sich bringt, ist letztlich die Menge der eingesetzten Schwefelsäure aus wirtschaftlichen Gründen nach oben hin beschränkt, da die resultierende Abfallsäuremenge recycliert oder weiterverarbeitet werden muss.

In einer bevorzugten Ausführungsform weist die Reaktionsmischung aus Acetoncyanhydrin (ACH) und Schwefelsäure in der ersten Reaktionsmischung eine Gesamtwassermenge im Bereich von 0,1 mol-% bis 20 mol-%, insbesondere 0,4 mol-% bis 10 mol-%, auf, bezogen auf das gesamte der ersten Reaktionsstufe zugeführte ACH. Bevorzugt wird in der ersten Reaktionsstufe Acetoncyanhydrin (ACH) eingesetzt, wobei das ACH bzw. die zugeführten ACH-Stoffströme (z.B. (1a) und/oder (1b)) einen Aceton-Gehalt von kleiner oder gleich 9.000 ppm, bevorzugt von kleiner oder gleich 1.000 ppm, aufweisen, bezogen auf die Gesamtmenge an ACH, die der ersten Reaktionsstufe zugeführt wird. Bevorzugt weist das eingesetzte ACH, bzw. die zugeführten ACH-Stoffströme (z.B. (1a) und/oder (1b)) einen ACH-Anteil von größer oder gleich 98 Gew.-%, besonders bevorzugt größer oder gleich 98,5 Gew.-%, insbesondere bevorzugt größer oder gleich 99 Gew.-%, auf, bezogen auf die zugeführten ACH-Stoffströme. Typischerweise enthält der zugeführte ACH-Stoffstrom (z.B. (1a) und/oder (1b)) 98,0 bis 99,8 Gew.-%, bevorzugt 98,3 bis 99,3 Gew.-%, Acetoncyanhydrin; 0,1 bis 1,5 Gew.-%, bevorzugt 0,2 bis 1 Gew.-%, Aceton, und 0,1 bis 1,5 Gew.-%, bevorzugt 0,3 bis 1 Gew.- %, Wasser, bezogen auf den ACH-Stoffstrom.

Bevorzugt wird in der ersten Reaktionsstufe Acetoncyanhydrin (ACH) eingesetzt, wobei das ACH bzw. die zugeführten ACH-Stoffströme (z.B. (1a) und/oder (1b)) einen Wassergehalt von 0,1 mol-% bis 10 mol-%, insbesondere 0,4 mol-% bis 5 mol-%, bezogen auf das in den zugeführten ACH-Stoffströme enthaltene ACH, aufweist.

Für die Durchführung der Amidierung (Hydrolyse) in der ersten Reaktionsstufe kann grundsätzlich jeder dem Fachmann für die Durchführung von Hydrolysereaktionen bekannte Reaktor verwendet werden, z.B. Rührkessel-Reaktoren und Schlaufenreaktoren beziehungsweise Kombinationen besagter Reaktoren. Alternativ können mehrere, ggf. parallel, bevorzugt jedoch in Reihe geschaltete Reaktoren verwendet werden. In einer möglichen Ausführungsform sind dabei 1 bis 5 Reaktoren in Reihe geschaltet, bevorzugt wird eine sequenzielle Anordnung von 2-3 Reaktoren verwendet.

Bevorzugt werden Schwefelsäure und ACH in der ersten Reaktionsstufe (Verfahrensschritt a) in einem molaren Verhältnis von Schwefelsäure zu ACH im Bereich von 1,2 bis 2; bevorzugt 1,25 bis 1,6; besonders bevorzugt von 1,4 bis 1,45 eingesetzt. Bevorzugt werden zwei oder mehrere Reaktoren I in der ersten Reaktionsstufe verwendet, wobei Schwefelsäure und ACH im ersten Reaktor I (Reaktor A) in einem molaren Verhältnis von Schwefelsäure zu ACH im Bereich von 1,6 bis 3; bevorzugt 1,7 bis 2,6; besonders bevorzugt von 1,8 bis 2,3; eingesetzt werden, und wobei Schwefelsäure und ACH im letzten Reaktor I (beispielsweise im zweiten Reaktor I, Reaktor D) in einem molaren Verhältnis von Schwefelsäure zu ACH im Bereich 1,2 bis 2,0; bevorzugt von 1,2 bis 1,8; insbesondere bevorzugt von 1,3 bis 1,7; eingesetzt werden. Als optimaler Kompromiss zwischen erzielbarer Ausbeute und Schwefelsäureverbrauch erweist sich ein molares Verhältnis von 1,25 bis 1,6, was letztlich unter wirtschaftlichen Optimierungsaspekten bewertet werden kann. Mehr Schwefelsäure erhöht die Kosten für diesen Einsatzstoff und den Aufwand für die Entsorgung der resultierenden Abfallsäuremischung, allerdings kann auch die auf ACH bezogene Ausbeute noch einmal leicht erhöht werden.

Die Umsetzung von Acetoncyanhydrin mit Schwefelsäure in der ersten Reaktionsstufe ist exotherm. Daher ist es vorteilhaft, die anfallende Reaktionswärme weitgehend oder zumindest teilweise abzuführen, beispielsweise mit Hilfe geeigneter Wärmetauscher, um eine verbesserte Ausbeute zu erhalten. Da mit sinkender Temperatur die Viskosität der Reaktionsmischung stark ansteigt und so Zirkulation, Durchfluss und Wärmeaustausch in den Reaktoren I (Reaktor A bzw. D) erschwert sind, ist eine zu starke Abkühlung allerdings in der Regel zu vermeiden. Darüber hinaus kann es bei niedrigen Temperaturen in der ersten Reaktionsmischung zu einer partiellen oder vollständigen Kristallisation von Inhaltsstoffen an den Wärmetauschern kommen, was zu Abrasion, beispielsweise in den Pumpengehäusen, Rohrleitungen und den Wärmetauscher-Rohren der Reaktoren I führen kann. Unterschreitet man die zulässige Amidierungstemperatur zu stark, so dass es zur Salzbildung und Fällung kommt, führt dies oftmals zur unerwünschten Abstellung der Anlage.

Zur Kühlung der Reaktorkreisläufe können grundsätzlich bekannte und geeignete Kühlmedien eingesetzt werden. Vorteilhaft ist der Einsatz von Kühlwasser. Typischerweise weist das Kühlmedium, insbesondere das Kühlwasser, eine Temperatur unterhalb der gewählten Verfahrensbedingungen auf. Vorteilhaft weist das Kühlmedium, insbesondere das Kühlwasser, eine Temperatur im Bereich von 20 bis 90°C, bevorzugt von 50 bis 90°C und besonders bevorzugt von 60 bis 70°C auf.

Zur Vermeidung von Unterschreitung des Kristallisationspunktes von Methacrylamid wird der Wärmetauscher typischerweise mit einem Warmwasser-Sekundärkreislauf betrieben. Hierbei sind Temperaturdifferenzen im produktseitigen Ein-/Austritt des Apparates von etwa 1 bis 20° C, insbesondere 2 bis 7°C bevorzugt.

Die Umsetzung von ACH und Schwefelsäure in ein oder mehreren Reaktoren I in einer ersten Reaktionsstufe (Amidierung) erfolgt bei einer Temperatur im Bereich von 70 bis 130°C, bevorzugt 70 bis 120°C, besonders bevorzugt von 85 bis 110°C. Wenn mehrere Reaktoren für die Amidierung eingesetzt werden, können die Temperaturen der diversen Reaktoren gleich oder unterschiedlich sein. Mit Blick auf unterschiedliche Stöchiometrien in den Reaktoren ist es bevorzugt, den ersten Reaktor mit höherem Schwefelsäure-Überschuss kälter zu betreiben als den oder die nachfolgenden Reaktoren. Oftmals wird die Amidierung in der erste Reaktionsstufe im Reaktor I oder in mehreren Reaktoren I bei Normaldruck durchgeführt.

Typischerweise kann die erste Reaktionsstufe (Amidierung) batchweise und/oder kontinuierlich durchgeführt werden. Bevorzugt wird die erste Reaktionsstufe kontinuierlich, beispielsweise in einem oder in mehreren Schlaufenreaktoren, durchgeführt. Geeignete Reaktoren und Verfahren sind beispielsweise in WO 2013/143812 beschrieben. Vorteilhaft kann die erste Reaktionsstufe in einer Kaskade von zwei oder mehreren Schlaufenreaktoren durchgeführt werden. Insbesondere bevorzugt erfolgt die Umsetzung der ersten Reaktionsstufe in einem oder in mehreren (bevorzugt zwei) Schlaufenreaktoren, wobei die Reaktionsmischung(en) bei einem Kreislaufverhältnis (Verhältnis aus Umwälzvolumenstrom zu Zulaufvolumenstrom) im Bereich von 5 bis 90, bevorzugt 10 bis 70 zur Reaktion gebracht werden. Alternativ können gerührte oder umgepumpte kontinuierliche Rührkessel-Reaktoren (CSTR-Reaktoren) verwendet werden, bzw. eine Kombination von CSTR Apparaten und Schlaufenreaktoren.

Die Verweilzeit im Schritt der Amidierung wird so ausgelegt, dass die Zeit ausreicht, um die Ausbeute von HIBA, SIBA, MAS und MASA zu maximieren. Typischerweise liegt die statische Verweilzeit in den Reaktoren I, insbesondere in den Schlaufenreaktoren I, im Bereich von 5 bis 35 Minuten, bevorzugt von 8 bis 20 Minuten.

Ein geeigneter Schlaufenreaktor weist bevorzugt die folgenden Elemente auf: eine oder mehrere Zugabestellen für ACH, eine oder mehrere Zugabestellen für Schwefelsäure, einen oder mehrere Gasabscheider, einen oder mehrere Wärmetauscher, einen oder mehrere Mischer, und eine Pumpe. Die Mischer sind häufig als statische Mischer ausgeführt.

Die Zugabe des ACH kann grundsätzlich an beliebiger Stelle in den einen oder in die mehreren Rektoren I (z.B. Schlaufenreaktoren) erfolgen. Es hat sich jedoch als vorteilhaft erwiesen, wenn die Zugabe des ACH an einer gut durchmischten Stelle erfolgt. Bevorzugt erfolgt die Zugabe des ACH in ein Mischelement, beispielsweise in einen Mischer mit beweglichen Teilen oder in einen statischen Mischer.

Die Zugabe der Schwefelsäure kann grundsätzlich an beliebiger Stelle in den einen oder in die mehreren Rektoren I (z.B. Schlaufenreaktoren) erfolgen (2). Bevorzugt erfolgt die Zugabe der Schwefelsäure vor der Zugabe des ACH. Besonders bevorzugt erfolgt die Zugabe der Schwefelsäure auf der Saugseite der jeweiligen Reaktorpumpe. Oftmals kann hierdurch die Pumpfähigkeit der gashaltigen Reaktionsmischung verbessert werden.

Die Reaktoren I (z.B. die Schlaufenreaktoren I) umfassen bevorzugt jeweils mindestens einen Gasabscheider. Typischerweise kann über den Gasabscheider einerseits kontinuierlich Produktstrom (erste Reaktionsmischung) entnommen werden, andererseits lassen sich gasförmige Nebenprodukte abtrennen und ausschleusen. Typischerweise bildet sich als gasförmiges Nebenprodukt hauptsächlich Kohlenstoffmonoxid. Bevorzugt wird der Abgasstrom der Reaktoren I mit dem Abgas aus dem Pufferbehälter/Abscheider I zusammengeführt.

In einer bevorzugten Ausführungsform umfasst die erste Reaktionsstufe die Umsetzung von Acetoncyanhydrin (ACH) und Schwefelsäure in mindestens zwei getrennten Reaktionszonen, bevorzugt in mindestens zwei getrennten Reaktoren, bevorzugt in mindestens zwei Schlaufenreaktoren (z.B. (A) bzw. (D)).

Bevorzugt erfolgt die Umsetzung von Acetoncyanhydrin (ACH) und Schwefelsäure in der Art, dass das Reaktionsvolumen auf mindestens zwei Reaktionszonen verteilt ist und die Gesamtmenge an ACH in die verschiedenen Reaktionszonen separat dosiert wird. Bevorzugt ist die Menge an ACH, die dem ersten Reaktor oder der ersten Reaktionszone zugeführt wird (z.B. (1a)), größer oder gleich der Mengen an ACH, die den nachfolgenden Reaktoren oder den nachfolgenden Reaktionszonen zugeführt werden (z.B. (1b)).

Vorzugsweise werden 50-90 Gew.-%, bevorzugt 60 bis 75 Gew.-%, des gesamten Volumenstroms an zugeführtem ACH, in den ersten Reaktor eingeführt (z.B. (1a)). Die verbleibende Menge an zugeführtem ACH wird in den zweiten Reaktor und gegebenenfalls in weitere Reaktoren eingeführt (z.B. (1b)). Typischerweise erfolgt eine Aufteilung der Gesamtmenge an ACH auf den ersten Reaktor I (z.B. (A)) und der zweiten Reaktor I (z.B. (B)) im Massenverhältnis erster Reaktor I: zweiter Reaktor I im Bereich von 70:30 bis 80:20, bevorzugt von etwa 75:25.

Bevorzugt ist das molare Verhältnis von zugegebener Schwefelsäure zu ACH im ersten Reaktor oder in der ersten Reaktionszone größer als das entsprechende molare Verhältnis in den nachfolgenden Reaktoren oder in den nachfolgenden Reaktionszonen.

Insbesondere bevorzugt umfasst die erste Reaktionsstufe die Umsetzung von (ACH) und Schwefelsäure in mindestens zwei getrennten Reaktoren, bevorzugt mindestens zwei Schlaufenreaktoren, wobei Schwefelsäure und ACH im ersten Reaktor in einem molaren Verhältnis von Schwefelsäure zu ACH im Bereich von 1,6 bis 3,0, bevorzugt 1,8 bis 3,0 eingesetzt werden, und wobei Schwefelsäure und ACH im zweiten Reaktor in einem molaren Verhältnis von Schwefelsäure zu ACH im Bereich von 1,2 bis 2,0, bevorzugt 1,3 bis 1,7, eingesetzt werden.

In einer besonders bevorzugten Ausführungsform erfolgt die Umsetzung in der ersten Reaktionsstufe in zwei oder mehreren Schlaufenreaktoren (z.B. (A) und (D)), wobei die Gesamtmenge an ACH in den ersten und mindestens einen weiteren Schlaufenreaktor dosiert wird. Insbesondere bevorzugt umfasst jeder Schlaufenreaktor mindestens eine Pumpe, einen mit Wasser als Medium gekühlten Wärmetauscher, eine Gastrennvorrichtung, mindestens eine Abgasleitung, verbunden mit der Gastrennvorrichtung, und mindestens eine Zuführleitung für ACH in flüssiger Form. Bevorzugt sind die mindestens zwei Schlaufenreaktoren derart miteinander verschaltet, dass die gesamte resultierende Reaktionsmischung des ersten Reaktors in die nachfolgenden Reaktoren geführt wird und die Reaktionsmischung in den nachfolgenden Reaktoren mit weiterem flüssigen ACH und optional weiteren Schwefelsäuremengen versetzt wird.

Der erste Schlaufenreaktor wird typischerweise bei einem Kreislaufverhältnis (Verhältnis aus Umwälzvolumenstrom zu Zulaufvolumenstrom) im Bereich von 5 bis 110, bevorzugt bei 10 bis 90, besonders bevorzugt bei 10 bis 70 betrieben. In einem nachfolgenden Schlaufenreaktor liegt das Kreislaufverhältnis vorzugsweise in einem Bereich von 5 bis 100, bevorzugt von 10 bis 90, besonders bevorzugt von 10 bis 70.

Typischerweise wird nach der ersten Reaktionsstufe (Amidierung) eine erste Reaktionsmischung (6) erhalten, welche 5 bis 35 Gew.-% Sulfoxyisobuttersäureamid (SIBA), 5 bis 25 Gew.-% Methacrylamid (MASA) sowie < 5 % Hydroxyisobuttersäureamid (HIBA), jeweils bezogen auf die gesamte Reaktionsmischung, gelöst in schwefelsaurer Reaktionsmatrix, enthält. Diese erste Reaktionsmischung wird bevorzugt mit Hilfe einer Austragspumpe (E) mit einem konstant geregelten Massenstrom in die zweite Reaktionsstufe gefördert. Der konstant geregelte Massenstrom ermöglicht insbesondere eine genaue Kontrolle der statischen Verweilzeit der Reaktionsmischung in der zweiten Reaktionsstufe (Verfahrensschritt (b), Konvertierung).

Bevorzugt wird die erste Reaktionsmischung resultierend aus der ersten Reaktionsstufe in einem konstant-geregeltem Massenstrom mittels einer Austragspumpe, ausgehend von mindestens einem Reaktor I durch mindestens einen Reaktor II gefördert.

### Zweite Reaktionsstufe (Konvertierung)

Das erfindungsgemäße Verfahren umfasst in Schritt (b) das Konvertieren der ersten Reaktionsmischung, umfassend das Erhitzen auf eine Temperatur im Bereich von 130 bis 200 °C, bevorzugt 130 bis 170 °C, besonders bevorzugt 140 bis 170 °C, weiterhin bevorzugt 140 bis 165 °C, in ein oder mehreren Reaktoren II (insbesondere auch als Wärmeumwandlungsapparatur bzw. Konvertierungsreaktoren bezeichnet) in einer zweiten Reaktionsstufe (Konvertierung), wobei eine zweite Reaktionsmischung (z.B. (7a)), enthaltend überwiegend Methacrylsäureamid (MASA) und Schwefelsäure, erhalten wird.

Erfindungsgemäß umfasst die Konvertierung, dass die Verweilzeit der Reaktionsmischung in der zweiten Reaktionsstufe im Bereich von 2 bis 15 min, bevorzugt von 2 bis 10 min, liegt. Typischerweise bezieht sich die angegebene Verweilzeit auf den gesamten Konvertierungsreaktor (Reaktor II). Typischerweise liegen die Verweilzeiten in allen Konvertierungsreaktoren in den angegebenen Bereichen. Bevorzugt beträgt die Verweilzeit in Vorheizer-Segment des Konvertierungsreaktors 0,1 bis 5 min; besonders bevorzugt 0,2 bis 2 min.

Erfindungsgemäß umfasst die Konvertierung, dass das Erhitzen in der zweiten Reaktionsstufe in einem oder mehreren Reaktoren II durchgeführt wird, wobei mindestens ein Reaktor II ein Vorheizer-Segment umfasst, welches ein oder mehrere lineare Rohrleitungselemente umfasst, die mittels eines räumlich von der Reaktionsmischung getrennten Heizmediums aufgeheizt werden, wobei die Reaktionsmischung um 10 bis 100°C, bevorzugt um 15 bis 80 °C, erhitzt wird. Typischerweise ist die Erhitzung um 10 bis 100°C so zu verstehen, dass eine entsprechende Temperaturerhöhung stattfindet, ausgehend von der Temperatur der Reaktionsmischung, welche in das jeweilige Vorheizer-Segment des Konvertierungsreaktors (Reaktor II) eintritt.

Erfindungsgemäß umfasst die Konvertierung, dass das Konvertieren in der zweiten Reaktionsstufe in einem oder mehreren Reaktoren II durchgeführt wird, wobei mindestens ein Reaktor II mindestens ein Verweilzeit-Segment umfasst, welches annähernd adiabatisch betrieben wird. Typischerweise umfasst das mindestens eine Verweilzeitsegment Rohrleitungselemente, die nicht mit Heizmedium begleitend beheizt sind und in denen somit insbesondere die Reaktionsmischung annähernd adiabatisch geführt wird.

Erfindungsgemäß umfasst die Konvertierung, dass das Vorheizer-Segment und/oder das Verweilzeit-Segment des Konvertierungsreaktors (Reaktor II) in einer Kombination aus linearen Rohrleitungselementen und Umlenkungen realisiert sind, wobei die linearen Rohrleitungselementen und die Umlenkungen über Reduzierflansche miteinander verbunden sind. Typischerweise stellt ein Reduzierflansch ein Rohrelement dar, das einen in Strömungsrichtung konvergent verlaufenden (verjüngenden) inneren Querschnitt aufweist.

Bevorzugt umfasst der mindestens eine Konvertierungsreaktors (Reaktor II) mindestens ein Vorheizer-Segment und mindestens ein Verweilzeit-Segment. Insbesondere bevorzugt umfassen alle Konvertierungsreaktoren (Reaktoren II) mindestens ein Vorheizer-Segment und mindestens ein Verweilzeit-Segment.

Besonders bevorzugt wird bereits die einen ersten Reaktor I (z.B. Reaktor A) verlassende Reaktionsmischung (z.B. (3)) einer solche Erhitzung (Konvertierung) unterzogen vor Eintritt in den oder die weiteren Schlaufenreaktor(en) I (z.B. Reaktor D). Diese Ausführungsform wird im Weiteren auch als Zwischenkonvertierung bezeichnet.

Typischerweise wird während der Konvertierung beim Erhitzen der ersten Reaktionsmischung auf eine Temperatur im Bereich von 130 bis 200 °C die Menge an MASA bzw. MASA·H₂SO₄ durch Dehydratisierung des HIBA bzw. durch Schwefelsäure-Eliminierung aus SIBA erhöht, wobei die erste Reaktionsmischung eine schwefelsaure Lösung enthaltend SIBA, HIBA und MASA, überwiegend jeweils in Form der Hydrogensulfate, darstellt.

Bevorzugt erfolgt das Erhitzen in der zweiten Reaktionsstufe (Konvertierung) über einen möglichst kurzen Zeitraum. Insbesondere erfolgt das Erhitzen in der zweiten Reaktionsstufe (i.e. Verweilzeit im Vorheizer-Segment des Konvertierungsreaktor) für einen Zeitraum von 0,1 bis 14 Minuten, bevorzugt 0.1 bis 5 Minuten, besonders bevorzugt 0.2 bis 2 Minuten. Üblicherweise wird die Erhitzung des Reaktionsgemisches als resultierende Mischung aus den Reaktoren 1 kürzer durchgeführt als die Konvertierung in den nahezu adiabaten Elementen selbst.

Insbesondere bevorzugt erfolgt die Konvertierung bei einer Temperatur im Bereich 130 bis 200 °C, bevorzugt 130 bis 170 °C, besonders bevorzugt 140 bis 170 °C, und einer Verweilzeit (i.e. gesamte Verweilzeit in Reaktor II oder Reaktoren II) im Bereich von 2 bis 15 min, bevorzugt von 2 bis 10 min.

Die Konvertierung kann grundsätzlich in bekannten Reaktoren durchgeführt werden, welche die Erzielung der genannten Temperaturen in den genannten Zeiträumen ermöglichen. Die Energiezufuhr kann hierbei in bekannter Weise, beispielsweise mittels Wasserdampfs, Heißwasser, geeigneten Wärmeträgern, elektrischer Energie oder elektromagnetischer Strahlung, wie Mikrowellenstrahlung, erfolgen. Bevorzugt wird die Konvertierung in der zweiten Reaktionsstufe in einem oder mehreren Wärmeumwandlungsapparaturen durchgeführt.

In einer bevorzugten Ausführungsform wird die Konvertierung in der zweiten Reaktionsstufe in einer Wärmeumwandlungsapparatur, umfassend eine zweistufige oder mehrstufige Anordnung von Rohrenleitungselementen, durchgeführt. Dabei können lineare und gebogene Rohleitungselemente miteinander kombiniert sein. Vorzugsweise liegen die mehrstufigen Rohre in einer gegenläufigen Anordnung dieser linearen Rohrleitungselemente, verbunden durch gebogene Rohrleitungselemente, vor, um den Flächenbedarf der Wärmeumwandlungsapparatur gering zu halten.

Im Verfahrensschritt der Konvertierung wird eine hohe Ausbeute der wertbildenden Komponenten MAS und MASA insbesondere dadurch erreicht, dass die eintretende Reaktionsmischung für eine definierte Zeit auf eine definierte Temperatur erhitzt wird. Dabei ist es vorteilhaft, die Reaktionsmischung möglichst rasch und innerhalb einer ersten definierten Zeit auf die benötigte Temperatur zu erwärmen (Vorheizen, Vorheizer-Segment) und dann bei dieser Temperatur für eine zweite definierte Zeit zu belassen (Verweilen, Verweilzeit-Segment). Die Temperaturführung beinhaltet somit bevorzugt einen ersten wärmezuführenden Schritt und einen nachfolgenden nahezu adiabatischen Schritt, bei dem von außen keine Wärme zugeführt wird und sich die Temperatur der Reaktionsmischung lediglich infolge nicht vermeidbarer Wärmeverluste verändert. Bevorzugt ist unter einer nahezu adiabaten Temperaturführung zu verstehen, dass sich die Temperatur der Reaktionsmischung im Verweilzeit-Schritt (bzw. im VerweilzeitSegment) um max. 10 K ändert.

Eine rasche Erwärmung im Sinne des erfindungsgemäßen Verfahrens ist typischerweise dann gewährleistet, wenn die erste, zum Vorwärmen aufgewendete Zeit (Verweilzeit im Vorheizer-Segment) nicht größer ist als die zweite zum Verweilen aufgewendete Zeit (Verweilzeit im Verweilzeit-Segment). Bevorzugt wird im erfindungsgemäßen Verfahren mindestens 60% der mittleren Verweilzeit der Konvertierung in Form der zweiten Zeit (Zeit im Verweilzeit-Segment) aufgewendet. Es ist für die Erzielung einer hohen Ausbeute insbesondere entscheidend, dass nur sehr wenige den Konvertierungsreaktor verlassenden Volumenelemente der Reaktionsmischung im Konvertierungsreaktor eine Verweilzeit aufweisen, die sich von der mittleren Verweilzeit unterscheidet. Der Fachmann spricht in diesem Sinne von einer schmalen beziehungsweise engen Verweilzeitverteilung.

Bevorzugt sind mindestens 60% der Verweilzeit, insbesondere der statischen Verweilzeit, von mindestens einer zweiten Reaktionsstufe (Konvertierung) in einem als Rohrleitung ausgeführten Verweilzeit-Segment realisiert.

Die Kontrolle und präzise Einstellung einer möglichst schmalen Verweilzeitverteilung in der Wärmeumwandlungsapparatur (Konvertierungsreaktor, Reaktor II) wird bevorzugt durch die Aufrechterhaltung eines pfropfenförmigen Strömungsprofils und die Minimierung von Rückvermischung in allen von der Reaktionsmischung durchströmten Rohrleitungselementen und Umlenkungen gewährleistet. Grundsätzlich kann die Wärmeumwandlungsapparatur (Konvertierungsreaktor II) theoretisch als ein gerades Rohr ausgestaltet sein. In einer bevorzugten, weniger Baufläche belegenden Ausführungsform enthält die Wärmeumwandlungsapparatur (Konvertierungsreaktor II) lineare Rohrleitungselemente und Umlenkungen. Zusätzliche Turbulenz erzeugende Einbauten, Torsionsschaufeln oder Leitbleche im inneren der Rohrleitungselemente, die im Dauerbetrieb einem Fouling unterliegen können und so den unterbrechungsfreien Betrieb des Prozesses gefährden, sind im Unterschied zum Stand der Technik (z.B. EP 0999200 B1) bevorzugt nicht erforderlich.

Das pfropfenförmige Strömungsprofil kann gemäß dem erfindungsgemäßen Verfahren insbesondere durch die geeignete Abstimmung von Rohrlängen und -durchmessern sowie der Abmessungen der Umlenkungen auf die in den Apparaten strömenden Reaktionsmischungen sichergestellt werden. Insbesondere wird durch das erfindungsgemäße Verfahren sichergestellt, dass Rückvermischungseffekte am Übergang zwischen linearen Rohrleitungselementen und Umlenkungen sowie innerhalb der Umlenkungen minimiert werden und damit eine Verbreiterung der Verweilzeitverteilung von erhitzen Reaktionsgemischen weitestgehend vermieden wird.

Die für die Konvertierung verwendete Wärmeumwandlungsapparatur setzt sich gemäß den genannten Erläuterungen bevorzugt aus einem ersten Vorheizer-Segment und einem nachfolgenden Verweilzeit-Segment zusammen. Das Vorheizer-Segment kann ein oder mehrere lineare Rohrleitungselemente umfassen, die mit einem räumlich von der sie durchströmenden Reaktionsmischung getrennten Heizmedium erhitzt werden, wobei die Reaktionsmischung um 10 bis 100 °C, bevorzugt um 15 bis 80 °C, erwärmt wird.

Das Leervolumen der linearen Rohrleitungselemente relativ zum Leervolumen der Umlenkungen in mindestens einem Vorheizer-Segment, bevorzugt in allen Vorheizer-Segmenten, entspricht einem Verhältnis (V_{Linear}/V_{Umlenkung}) im Bereich von 2,0 bis 75,0; bevorzugt im Bereich von 4,0 bis 75,0; insbesondere bevorzugt im Bereich von 5,0 bis 50,0; weiterhin bevorzugt im Bereich von 5,0 bis 20,0.

In einer bevorzugten Ausführungsform umfasst mindestens ein Vorheizer-Segment lineare Rohrleitungselemente und Umlenkungen, wobei die linearen Rohrleitungselemente des Vorheizer-Segments 1 bis 50 separate gerade Rohrleitungen umfassen, die mit einem Heizmedium sekundär beheizt werden und parallel zueinander angeordnet sind, und wobei die Umlenkungen, welche die linearen Rohrleitungselemente miteinander verbinden, mindestens eine Rohrleitung umfassen.

In einer bevorzugten Ausführungsform sind im Vorheizer-Segment die linearen Rohrleitungselemente in ein oder mehreren geraden Rohrleitungen ausgeführt, die einen inneren Durchmesser im Bereich von 8 mm bis 200 mm aufweisen, wobei die Rohrleitungen der linearen Rohrleitungselemente im Vorheizer-Segment einen kleineren inneren Durchmesser aufweisen können als die Rohrleitungen der linearen Rohrleitungselemente im Verweilzeit-Segment.

Bevorzugt umfasst das Vorheizer-Segment 1 bis 50, bevorzugt 2 bis 20, separate lineare Rohleitungselemente mit einem inneren Durchmesser von jeweils 8 mm bis 200 mm, bevorzugt von 12 mm bis 160 mm, wobei bevorzugt alle linearen Rohrleitungselemente den gleichen Durchmesser aufweisen. Bei Durchströmung mit der Reaktionsmischung resultiert in den linearen Rohrleitungselementen bevorzugt eine mittlere Strömungsgeschwindigkeit von mindestens 0,2 m/s.

Vorzugsweise werden das eine oder die mehreren beheizten linearen Rohrleitungselemente des Vorheizer-Segments in einer Halterung oder einem Gestell parallel zueinander gestapelt angeordnet, um den Platzbedarf des Vorheizer-Segments gering zu halten. Die Anordnung wird typischerweise als Rohrbündel ausgestaltet. Dabei können die mehreren linearen Rohrleitungselemente parallel (im Gleichstrom) oder seriell (abwechselnd im Gegenstrom) von der Reaktionsmischung durchströmt werden, wobei ein serieller Betrieb bevorzugt wird. Die prozessseitige Verbindung der seriell durchströmten linearen Rohrleitungselemente kann durch bekannte Vorrichtungen, wie z.B. Hauben oder Umlenkungen, erfolgen.

Es hat sich herausgestellt, dass eine solche konstruktive Ausführung der Umlenkungen bevorzugt vorteilhaft für die Erzielung hoher Ausbeuten ist, bei der die Umlenkungen möglichst totraumfrei und mittels zweier je um 90° gebogenen Rohleitungselemente oder mittels eines um 180° gebogenen Rohleitungselements realisiert sind, und bei der die Umlenkung einen einzigen inneren Querschnitt aufweist. Bevorzugt wird die Umlenkung mittels zweier um 90° gebogener Rohrleitungselemente realisiert, die untereinander mit einem zweiten, bevorzugt nicht beheizten, linearen Rohrleitungselement verbunden sind, und wobei das gesamte Leervolumen aller linearen Rohrleitungselemente des VorheizerSegments relativ zum Leervolumen der Umlenkungen des Vorheizersegments einem Verhältnis im Bereich von 2,0 bis 75,0, bevorzugt im Bereich von 4,0 bis 75,0; insbesondere bevorzugt im Bereich von 5,0 bis 50,0; weiterhin bevorzugt im Bereich von 5,0 bis 20,0 entspricht. Insbesondere umfasst das Leervolumen der Umlenkung jeweils das Volumen der zwei um 90° gebogener Rohrleitungselemente und das Volumen des dazwischen liegenden linearen Rohrleitungselement.

In einer bevorzugten Ausführungsform sind die linearen Rohrleitungselemente im Vorheizer-Segment und/oder im Verweilzeit-Segment des mindestens einen Reaktors II in parallelen Rohrbündeln ausgeführt und diese Rohrbündel sind mittels mindestens einer Umlenkung verbunden, wobei die Umlenkung als Rohrleitung ausgestaltet ist, welches einen ersten Abschnitt, der einen in Strömungsrichtung konvergent verlaufenden inneren Querschnitt aufweist, einen zweiten Abschnitt, der eine im Wesentlichen konstante innere Querschnittsfläche aufweist, und einen dritten Abschnitt, der einen in Strömungsrichtung divergent verlaufenden inneren Querschnitt aufweist, umfasst. Bevorzugt kann der zweite Abschnitt der Umlenkungen, der eine im Wesentlichen konstante innere Querschnittsfläche aufweist, als Rohrbogen, bevorzugt als 90° oder 180° Rohrbogen, ausgestaltet sein.

Bevorzugt sind die Umlenkungen (beispielsweise die Umlenkungen im Vorheizer-Segment und/oder im Verweilzeitsegment) aus Rohrleitungselementen gebildet, die eine Biegung von 90° bis 180°aufweisen. Insbesondere bevorzugt weisen die Umlenkungen etwa 180 ° auf.

Im Sinne der präzisen Kontrolle der Verweilzeitverteilung im gesamten Vorheizer-Segment ist es insbesondere vorteilhaft, den inneren Durchmesser der Umlenkungen so zu bemessen, dass die Rückvermischung am Übergang zwischen den linearen Rohrleitungselementen und den Umlenkungen und innerhalb der Umlenkungen selbst minimiert und eine hohe lineare Strömungsgeschwindigkeit aufrechterhalten wird. Bevorzugt wird dies sichergestellt durch Einhaltung eines minimalen Verhältnis der Querschnittsfläche der mit Reaktionsgemisch durchströmten linearen beheizten Rohrleitungselemente zum freien Querschnitt der Umlenkungen (insbesondere dem freien Querschnitt des zweiten Abschnitts der Umlenkung, der eine im Wesentlichen konstante innere Querschnittsfläche aufweist) (A_{Rohr}/ A_{Umlenkung}), von mindestens 0,1 m²/m². Bevorzugt wird ein Querschnittsverhältnis von 0,8 m²/m² eingehalten, entsprechend einer Strömungsgeschwindigkeit in den Umlenkungen von > 0,8 m/s.

Als Heizmedium kann jedes dem Fachmann bekannte Heizmedium, z.B. ein thermisch belastbares Öl, ein Salzbad, elektromagnetische Strahlung, eine elektrische Beheizung, überhitztes Wasser oder Wasserdampf verwendet werden. Bevorzugt wird Sattdampf als Heizmedium verwendet.

Das Verweilzeit-Segment kann analog dem Vorheizer-Segment aus einem oder mehreren Rohren bestehen, die jedoch im Unterschied zu diesem unbeheizt sind. Bevorzugt sind das eine oder die mehreren Rohre des Verweilzeit-Segments in einer Halterung oder einem Gestell parallel zueinander gestapelt angeordnet, um den Platzbedarf des Verweilzeit-Segments gering zu halten. Die Anordnung wird ebenfalls typischerweise als Rohrbündel ausgestaltet. Dabei können die mehreren Rohre parallel (im Gleichstrom) oder seriell (abwechselnd im Gegenstrom) von der Reaktionsmischung durchströmt werden, wobei ein serieller Betrieb bevorzugt wird. Die prozessseitige Verbindung der seriell durchströmten Rohre kann durch jede dem Fachmann bekannte Art von Verbindung ausgeführt sein; bevorzugt werden die bereits beim Vorheizer-Segment als Umlenkung beschriebene Kombination von Rohrleitungselementen verwendet. Bevorzugt weisen die linearen Rohrleitungselemente des Verweilzeit-Segments einen inneren Durchmesser im Bereich von 8 mm und 200 mm auf, wobei die linearen und gebogenen Rohrleitungselemente des Verweilzeit-Segments einen größeren inneren Durchmesser aufweisen können als die entsprechenden Rohrleitungselemente des VorheizerSegments.

Wie durch CFD-Simulationen gezeigt werden konnte, ist in konstruktiver Hinsicht die Einhaltung bestimmter Geometrien und Abmessungsverhältnisse der die Umlenkungen bildenden gebogenen Rohrleitungselemente und der zwischen den Umlenkungen vorgesehenen zweiten linearen Rohrleitungselemente bevorzugt für eine Minimierung von Rückvermischungs-Effekten, die Gewährleistung einer pfropfenförmigen Strömung und somit für eine präzise Einstellung und Kontrolle der Verweilzeiten im Vorheizer- bzw. Verweilzeitsegment. Die Einhaltung dieser konstruktiven Parameter gestattet dabei überraschenderweise auch den Fortbestand der genannten Vorteile bei einem Teillastbetrieb des beschriebenen Verfahrens, also bei Strömungsgeschwindigkeiten, die geringer sind als im Falle der Nennlast. Somit kann gezeigt werden, dass die erzielte Ausbeute im Teillastbereich ähnlich oder identisch ist, wie die erzielte Ausbeute beim Auslegungspunkt für Voll-Last. Als Teil-Last werden Lastzustände der Anlage bezeichnet, bei denen das Feedgemisch gegenüber der Voll-Lastfahrweise allgemein reduziert ist. Eine Teil-Last der Anlage umfasst die Reduktion der Feedmengen um bis zu 70%, bevorzugt um bis zu 50%.

Zur Minimierung von Rückvermischungseffekten in den die linearen Rohleitungselemente verbindenden Umlenkungen wird der innere Durchmesser der Umlenkungen bevorzugt so gewählt, dass innerhalb der Umlenkungen eine mittlere Geschwindigkeit des Reaktionsmediums von 0,2 bis 3 m/sec, bevorzugt von 0,2 bis 2 m/sec, insbesondere bevorzugt von 0,5 bis 2 m/sec, eingestellt wird und die Abweichung der lokalen Geschwindigkeit an jeder Stelle innerhalb der Umlenkungen von der mittleren Fördergeschwindigkeit nicht mehr als 30% beträgt. Bevorzugt wird innerhalb der Umlenkungen eine mittlere Geschwindigkeit der Reaktionsmischung von 0,2 bis 3 m/sec, bevorzugt von 0,2 bis 2 m/sec, insbesondere bevorzugt von 0,5 bis 2 m/sec, eingestellt. Bevorzugt wird innerhalb der Umlenkungen eine mittlere Geschwindigkeit der Reaktionsmischung von 0,2 bis 3 m/sec, bevorzugt von 0,2 bis 2 m/sec, insbesondere bevorzugt von 0,5 bis 2 m/sec, eingestellt und die Abweichung der lokalen Geschwindigkeit an jeder Stelle innerhalb der Umlenkungen von der mittleren Fördergeschwindigkeit beträgt kleiner oder gleich 30%.

Die für die Konvertierungsreaktoren, umfassend mindestens ein Vorheizer-Segment und mindestens ein Verweilzeit-Segment, verwendete Konstruktion umfasst bevorzugt lineare Rohrleitungselemente und Umlenkungen. Bevorzugt können die Rohrleitungen und Rohrleitungselemente des Konvertierungsreaktors, aus jedem dem Fachmann bekannten Werkstoff bestehen, der gegen starke Säuren unter den beschriebenen Reaktionsbedingungen und -temperaturen beständig ist. Geeignete Materialien für die Vorheizer-Segmente umfassen, sind jedoch nicht beschränkt auf, Hastelloy-Legierungen, Tantal-Legierungen sowie Siliciumcarbid enthaltende Keramiken. Die Verweilzeit-Segmente können aus kostengünstigeren Materialien wie Stahl/PTFE-Kombinationen, Stahl/Emaille-Kombinationen oder Stahl/Keramik-Kombinationen konstruiert werden. Typischerweise sind die Vorheizer-Segmente aus Materialien hoher Wärmeleitfähigkeit konstruiert, während sich die Materialien der Verweilzeit-Segmente durch eine geringere thermische Leitfähigkeit auszeichnen.

In einer bevorzugten Ausführungsform sind an mindestens einem Rohrbündelwärmetauscher Reduzierflansche im Übergang der linearen Rohrleitungselemente zu den Umlenkungen installiert, welche insbesondere als totraumarme Reduzierflansche ausgeführt sind, so dass ein Flächenverhältnis zwischen der freien Querschnittsfläche der mit Reaktionsmischung durchströmten linearen beheizten Rohrleitungselemente zum freien Querschnitt der Umlenkungen (A_{Rohr} / A_{Umlenkung}), von mindestens 0,1 m²/m² realisiert ist. Insbesondere bezieht sich die freie Querschnittsfläche der linearen beheizten Rohrleitungselemente auf die Summe der freien Querschnittsflächen aller Rohrleitungen der linearen Rohrleitungen (bzw. Rohrleitungsbündeln).

In einer bevorzugten Ausführungsform ist an mindestens einem der Reduzierflansche ein 180° Rohrbogen installiert, welcher ein Verhältnis der freien Querschnittsfläche der innerhalb des Wärmtauschers vorliegenden linearen Rohrleitungselemente zur freien Querschnittsfläche der Umlenkungen von mindestens 0,8 m²/m² aufweist und damit mindestens eine Strömungsgeschwindigkeit > 0,8 m/s innerhalb der Umlenkungen realisiert.

Die Verweilzeit im der zweiten Reaktionsstufe (Verfahrensschritt (b), Konvertierung), umfassend die Verweilzeit im Vorheizer-Segment und die Verweilzeit im VerweilzeitSegment kann insbesondere abhängig von den Reaktionsmischungen und Temperaturen variieren. Es hat sich herausgestellt, dass zur Vervollständigung der gewünschten Reaktionen (HIBA --> MASA sowie SIBA → MASA) einerseits und zur Minimierung der die Ausbeute vermindernden Folgereaktionen andererseits ein definierter Verweilzeitbereich vorteilhaft ist. Dieser Verweilzeitbereich beträgt typischerweise 2 bis 15 Minuten.

Insbesondere ist der Konvertierungsreaktor in der Art ausgestaltet, dass die folgenden Faktoren berücksichtigt werden: Aufrechterhalten der pfropfenförmigen Strömung, die gewünschte Verweilzeit, die Flussrate, das Reaktionsgemisch und die Temperaturführung innerhalb der Konverter.

In einer bevorzugten Ausführungsform (Zwischenkonvertierung) findet bei der Durchführung der ersten Reaktionsstufe (Amidierung) und der zweiten Reaktionsstufe (Konvertierung) mindestens eine Zwischenkonvertierung zwischen mindestens zwei ersten Reaktionsstufen (Amidierung) statt, wobei die mindestens zwei Reaktoren II der Konvertierung durch mindestens einen Rohrbündelwärmetauscher als Vorheizer-Segment, verbunden mit mindestens einer Verweilzeitstrecke als Verweilzeit-Segment, realisiert sind.

In einer bevorzugten Ausführungsform (Zwischenkonvertierung) werden mindestens zwei Reaktoren I (Amidierung) verwendet werden, wobei mindestens ein Reaktor II umfassend mindestens ein Vorheizer- und/oder Verweilzeit-Segment zwischen den Reaktoren I sowie mindestens ein Reaktor II umfassend mindestens ein Vorheizer- und/oder VerweilzeitSegment am Austritt der ersten Reaktionsstufe (Amidierung), zum Einsatz kommen.

Bei der Ausführungsform mit Zwischenkonvertierung kann ein Zwischenkühler verwendet werden, um die heiße Konvertierungsmischung vor Rückgabe in die Amidierung zu kühlen. Der Kühler kann aber entfallen, wenn innerhalb der Amidierung die Kühlvorrichtungen ausreichend dimensioniert sind, um die Konvertierungswärme zu reduzieren.

Bevorzugt ist in mindestens einem Reaktor II mindestens ein Vorheizer-Segment als Rohrbündelwärmetauscher ausgestaltet, wobei der Rohrbündelwärmetauscher rohrseitig mit der Reaktionsmischung durchströmt wird und durch die freie Querschnittsfläche der verwendeten Rohre des Rohrbündelwärmetauschers eine mittlere Strömungsgeschwindigkeit von mindestens 0,2 m/s in den Rohren gewährleistet ist.

Die für die Konvertierung verwendete Wärmeumwandlungsapparatur kann weiterhin bevorzugt mit einem oder mehreren Gasabscheidern kombiniert werden. So ist es beispielsweise möglich, die Reaktionsmischung nach Verlassen des Vorheizer-Segments der Wärmeumwandlungsapparatur (Reaktor II) und/oder nach Verlassen des VerweilzeitSegments der Wärmeumwandlungsapparatur (Reaktor II) durch einen Gasabscheider zu führen. Dabei können insbesondere gasförmige Nebenprodukte aus der Reaktionsmischung abgetrennt werden.

In einer bevorzugten Ausführungsform wird die in Schritt b erhaltene zweite Reaktionsmischung, enthaltend überwiegend Methacrylsäureamid und Schwefelsäure, auf eine Temperatur unterhalb von 120 °C, bevorzugt auf eine Temperatur im Bereich von 90 bis 120 °C, bevorzugt auf eine Temperatur im Bereich von 90 bis 119 °C, abgekühlt, beispielsweise in einem Kühler mit einem Kühlmedium mit einer Temperatur im Bereich von 60 bis 100°C. Bevorzugt wird die in Schritt b erhaltene zweite Reaktionsmischung, bevorzugt nach der Abkühlung, in einem Zwischenbehälter (auch als Pufferbehälter bezeichnet, z.B. (I)) zwischengepuffert, bevor die Reaktionsmischung in die dritte Reaktionsstufe geführt wird. Bevorzugt liegt die Verweilzeit im Zwischenbehälter (Pufferbehälter) (z.B. (I)) im Bereich von 5 bis 60 min.

Typischerweise wird die erste Reaktionsmischung, erhalten in der ersten Reaktionsstufe, vollständig in den Reaktor II der zweiten Reaktionsstufe geführt. Bevorzugt wird nach jedem als Schlaufenreaktor ausgeführten Reaktor I die austretende erste Reaktionsmischung in einen als Wärmeumwandlungsapparatur ausgeführten Reaktor II geführt. Die aus einem solchen Reaktor II austretende zweite Reaktionsmischung kann ihrerseits wieder in einen weiteren, nachfolgenden Reaktor I geführt oder, in einem Kühler (z.B. (H)) gekühlt und anschließend in einem Pufferbehälter (z.B. (I)) gelagert werden, bevor sie als Strom (z.B. (8)) der dritten Reaktionsstufe (c) (Veresterung oder Hydrolyse) zugeführt wird.

Die aus der Konvertierung austretende, noch heiße Reaktionsmischung (z.B. (7a)) wird vor Eintritt in den Pufferbehälter (z.B. (I)) bevorzugt in einem Kühler (z.B. (H)) gekühlt unter Erhalt eines gekühlten Stroms (z.B. (7b)), um eine definierte Verweilzeit der den letzten Konvertierungsreaktor verlassenden Reaktionsmischung bei hohen Temperaturen zu gewährleisten und ausbeutevermindernde Folgereaktionen der in der Konvertierung gebildeten Wertprodukte MAS und MASA zu unterbinden, und wobei gasförmige Nebenprodukte zumindest teilweise von der zweiten Reaktionsmischung abgetrennt werden können. Der Pufferbehälter (z.B. (I)) erfüllt dabei zum einen die Funktion, einen gleichmäßigen Zustrom zur nachfolgenden dritten Reaktionsstufe (Veresterung oder Hydrolyse) zu gewährleisten. Zum anderen gestattet er insbesondere eine weitere Entgasung der gekühlten zweiten Reaktionsmischung und fungiert somit als Gasabscheider. Typischerweise wird die entgaste zweite Reaktionsmischung vollständig in die dritte Reaktionsstufe (Veresterung oder Hydrolyse) geführt. Bevorzugt wird das Abgas (9), welches im Pufferbehälter (z.B. (I)) nach der Konvertierung erhalten wird, vollständig oder teilweise aus dem Verfahren ausgeschleust. Weiterhin bevorzugt wird das Abgas, welches im Gasabscheider nach der Konvertierung erhalten wird, vollständig oder teilweise in die dritte Reaktionsstufe (Veresterung oder Hydrolyse) geführt.

Der Kühler (z.B. (H)) kann bevorzugt als mindestens ein Wärmetauscher ausgeführt sein, der verwendet wird, um die Temperatur der Reaktionsmischung (z.B. (7a)) vor der Veresterungsreaktion zu senken und einen Abbau der Wertprodukte zu verhindern. Der Kühler (z.B. (H)) kann typischerweise in jeder dem Fachmann bekannten Art von Wärmetauscher ausgeführt sein, die bei der beschriebenen Temperatur und unter den stark sauren Bedingungen beständig ist. Geeignete Wärmetauscher umfassen Platte und Rahmen, Platte und Rippe, Spirale und Rohr. Geeignete Konstruktionsmaterialien können umfassen, sind jedoch nicht beschränkt auf, Hastelloy B, Hastelloy B-2, Hastelloy B-3, Siliciumcarbid und Tantallegierungen, insbesondere auch Werkstoffe und Werkstoffkombinationen, die Emaille, andere keramische Materialien und chemisch und thermisch beständige Kunststoffe enthalten.

Zur Kühlung der zweiten Reaktionsmischung können grundsätzlich bekannte und geeignete Kühlmedien eingesetzt werden. Vorteilhaft ist der Einsatz von Kühlwasser. Typischerweise weist das Kühlmedium, insbesondere das Kühlwasser, eine Temperatur unterhalb der gewählten Verfahrensbedingungen auf. Vorteilhaft weist das Kühlmedium, insbesondere das Kühlwasser, eine Temperatur im Bereich von 30 bis 120°C, bevorzugt von 50 bis 110°C und besonders bevorzugt von 60 bis 100°C auf. Die Austrittstemperatur der den Kühler (z.B. (H)) verlassenden gekühlten Reaktionsmischung (z.B. (7b)) und somit die Eintrittstemperatur in den nachfolgenden Reaktionsschritt (dritte Reaktionsstufe (Veresterung oder Hydrolyse) (c)) liegt bevorzugt unterhalb der maximalen im Konvertierungsreaktor auftretenden Temperatur, typischerweise im Bereich von 70 °C bis 110 °C.

### Dritte Reaktionsstufe (Veresterung oder Hydrolyse)

Das erfindungsgemäße Verfahren umfasst in Schritt (c) die Umsetzung der zweiten Reaktionsmischung, enthaltend überwiegend MASA, mit Wasser und optional Methanol, in ein oder mehreren Reaktoren III in einer dritten Reaktionsstufe (Veresterung oder Hydrolyse), wobei eine dritte Reaktionsmischung, enthaltend Methacrylsäure und/oder Methylmethacrylat, erhalten wird.

In einer bevorzugten Ausführungsform (Veresterung) erfolgt die Umsetzung der zweiten Reaktionsmischung, enthaltend überwiegend MASA, mit Wasser und Methanol, wobei eine dritte Reaktionsmischung, enthaltend Methylmethacrylat, erhalten wird. Die Bedingungen der Veresterung im großtechnischen Maßstab sind dem Fachmann bekannt und beispielsweise in US 5,393,918 beschrieben.

In einer weiteren Ausführungsform (Hydrolyse) erfolgt die Umsetzung der zweiten Reaktionsmischung, enthaltend überwiegend MASA, mit Wasser, wobei eine dritte Reaktionsmischung, enthaltend Methacrylsäure, erhalten wird. Die Bedingungen der Hydrolyse von Methylacrylamid zu Methacrylsäure und die Verfahren zur Aufbereitung im großtechnischen Maßstab sind dem Fachmann bekannt und beispielsweise in DE 10 2008 000 787 A1 und EP 2 292 580 beschrieben.

Die Umsetzung in der dritten Reaktionsstufe (Veresterung oder Hydrolyse) wird bevorzugt in einem oder mehreren geeigneten Reaktoren, beispielsweise in beheizten Kesseln durchgeführt. Insbesondere können mit Wasserdampf beheizte Kessel verwendet werden. In einer bevorzugten Ausführungsform erfolgt die Veresterung in zwei oder mehreren, beispielsweise drei oder vier, aufeinanderfolgenden Kesseln (Kesselkaskade).

Typischerweise wird die Veresterung bei Temperaturen im Bereich von 100 bis 180 °C, bevorzugt von 100 bis 150 °C, bei Drücken von bis zu 7 bara, bevorzugt bei Drücken bis zu 2 bara, und unter Verwendung von Schwefelsäure als Katalysator durchgeführt.

Bevorzugt erfolgt die Umsetzung der zweiten Reaktionsmischung mit einem Überschuss an Methanol und Wasser. Die Zugabe der zweiten Reaktionsmischung, enthaltend überwiegend Methacrylamid, und die Zugabe von Alkohol erfolgen bevorzugt in der Art, dass sich ein molares Verhältnis von Methacrylamid zu Alkohol im Bereich von 1:1,0 bis 1:1,6 ergibt. In einer bevorzugten Ausführungsform erfolgt die Umsetzung in der dritten Reaktionsstufe in zwei oder mehreren Reaktoren III, wobei sich im ersten Reaktor III ein molares Verhältnis von Methacrylamid zu Alkohol im Bereich von 1:0,7 bis 1:1,4 ergibt (bevorzugt 1:1.0 bis 1:1.4), und wobei sich im zweiten und den möglichen nachfolgenden Reaktoren III ein molares Verhältnis von Methacrylamid zu Alkohol im Bereich von 1:1,05 bis 1:1,3 ergibt.

Bevorzugt setzt sich der in die dritte Reaktionsstufe (Veresterung oder Hydrolyse) zugeführte Alkohol zusammen aus frisch ins Verfahren zugeführtem Alkohol (Frischalkohol) und aus Alkohol, welcher in zurückgeführten Strömen (Recyclingströmen) des erfindungsgemäßen Verfahrens enthalten ist. Es ist zudem möglich, Methanol im erfindungsgemäßen Verfahren zu verwenden, welcher in Recyclingströmen aus nachgeschalteten Downstream-Prozessen enthalten ist.

Typischerweise erfolgt die Zugabe an Wasser in den Reaktor III oder in die Reaktoren III der dritten Reaktionsstufe derart, dass die Konzentration an Wasser im Bereich von 10 bis 30 Gew.-%, bevorzugt 15 bis 25 Gew.-%, bezogen jeweils auf die gesamte Reaktionsmischung im Reaktor III, liegt.

Grundsätzlich kann das in die dritte Reaktionsstufe (Veresterung oder Hydrolyse) zugeführte Wasser aus einer beliebigen Quelle stammen und verschiedene organische Verbindungen enthalten, sofern keine Verbindungen enthalten sind, welche die Veresterung oder die nachfolgenden Verfahrensstufen nachteilig beeinflussen. Bevorzugt stammt das in die dritte Reaktionsstufe zugeführte Wasser aus zurückgeführten Strömen (Recyclingströmen) des erfindungsgemäßen Verfahrens, beispielsweise aus der Aufreinigung des Methylmethacrylats oder der Methacrylsäure. Es ist zudem möglich Frischwasser, insbesondere vollentsalztes Wasser oder Brunnenwasser, in die dritte Reaktionsstufe (Veresterung oder Hydrolyse) zu zuführen.

Die Veresterung mit Methanol liefert typischerweise eine dritte Reaktionsmischung enthaltend Methylmethacrylat (MMA), Hydroxyisobuttersäuremethylester (HIBSM) und weitere oben beschriebene Nebenprodukte sowie signifikante Mengen Wasser und nicht umgesetzte Methanol.

In einer bevorzugten Ausführungsform erfolgt die Veresterung in zwei oder mehreren (insbesondere drei oder vier) aufeinander folgenden Kesseln (Kesselkaskade), wobei der flüssige Überlauf und die gasförmigen Produkte aus dem ersten Kessel in den zweiten Kessel geführt werden. Typischerweise wird mit möglichen nachfolgenden Kesseln in entsprechender Weise verfahren. Insbesondere kann durch eine solche Fahrweise die Schaumbildung in den Kesseln reduziert werden. Im zweiten Kessel und in den möglichen nachfolgenden Kesseln kann ebenfalls eine Zugabe von Alkohol erfolgen. Bevorzugt ist hierbei die Menge an zugegeben Alkohol um mindestens 10 % kleiner im Vergleich zum vorhergehenden Kessel. Die Konzentrationen an Wasser in den verschiedenen Kesseln können sich typischerweise voneinander unterscheiden. Typischerweise beträgt die Temperatur der in den ersten Kessel eingespeisten zweiten Reaktionsmischung im Bereich von 100 bis 180 °C. Typischerweise liegt die Temperatur im ersten Kessel im Bereich von 90 bis 180 °C und die Temperatur im zweiten und in den möglichen nachfolgenden Kesseln im Bereich von 100 bis 150 °C.

Typischerweise erfolgt die Hydrolyse, indem die zweite Reaktionsmischung mit Wasser zur Reaktion gebracht wird, wobei MASA zu MAS hydrolysiert wird, und das entstehende Ammoniak als Ammoniumhydrogensulfat anfällt. Eine Vielzahl solcher Verfahren sind im Stand der Technik beschrieben, zum Beispiel in US 7,253,307. Beispielsweise wird MASA mit Wasser bei moderaten Druck und Temperaturen zwischen 50° bis 210° in Gegenwart überstöchiometrischer Mengen an Schwefelsäure in Rührkessel-Reaktoren oder Kreislaufreaktoren zu Methacrylsäure umgesetzt.

Typischerweise erfolgt die Umsetzung der zweiten Reaktionsmischung mit Wasser (Hydrolyse) chargenweise oder kontinuierlich, beispielsweise in einem Rohrreaktor oder einem Rührkesselreaktor. Die Umsetzung der zweiten Reaktionsmischung mit Wasser (Hydrolyse) kann beispielsweise in einem oder mehreren geeigneten Reaktoren III, beispielsweise in beheizten Kesseln oder Rohrreaktoren bei Temperaturen im Bereich von 90 bis 130 °C, bevorzugt von 100 bis 125 °C, durchgeführt werden. Bevorzugt erfolgt die Umsetzung der zweiten Reaktionsmischung mit einem Überschuss an Wasser in der Art, dass sich ein molares Verhältnis von Wasser zu Methacrylsäureamid im Bereich von 1 bis 8, bevorzugt 3 bis 6 ergibt.

Die Hydrolyse mit Wasser liefert typischerweise eine dritte Reaktionsmischung enthaltend Methacrylsäure, Hydroxyisobuttersäure (HIBS) und weitere oben beschriebene Nebenprodukte sowie signifikante Mengen Wasser.

### Aufarbeitung

Typischerweise umfasst das Verfahren die Abtrennung von Methylmethacrylat und/oder Methacrylsäure aus der dritten Reaktionsmischung (Aufarbeitung) erhalten aus der dritten Reaktionsstufe (Veresterung oder Hydrolyse). Hierbei können insbesondere die dem Fachmann bekannten Trennschritte zum Einsatz kommen, insbesondere Rektifikations-, Extraktions-, Strippungs- und/oder Phasentrennschritte. Bevorzugt umfasst die Abtrennung von Methylmethacrylat und/oder Methacrylsäure aus der dritten Reaktionsmischung mindestens einen Destillationsschritt, bevorzugt mindestens zwei Destillationsschritte.

Bevorzugt umfasst die Aufarbeitung die Vorreinigung der dritten Reaktionsmischung, welche in der Veresterung erhalten wird. Insbesondere umfasst die Vorreinigung mindestens einen Destillationsschritt, mindestens einen Phasentrennungsschritt und mindestens einen Extraktionsschritt. Bevorzugt umfasst die Vorreinigung mindestens einen, bevorzugt mindestens zwei Destillationsschritte. Insbesondere wird in der Vorreinigung ein MMA-Rohprodukt und/oder MAS-Rohprodukt erhalten, welches eine Reinheit im Bereich von mindestens 85 Gew.-% aufweist.

Bevorzugt umfasst die Aufarbeitung die Feinreinigung des Rohproduktes, wobei die Feinreinigung mindestens einen, bevorzugt mindestens zwei Destillationsschritte umfasst. Insbesondere wird in der Feinreinigung ein MMA-Rohprodukt und/oder MAS-Rohprodukt erhalten, welches eine Reinheit von mindestens 99.0 Gew.-% aufweist.

In einer weiteren Ausführungsform umfasst die Aufarbeitung die Abtrennung von Methacrylsäure (MAS) aus der dritten Reaktionsmischung. Einzelheiten zur Aufarbeitung zur Gewinnung von MAS sind in DE 10 2008 000 787 A1 beschrieben.

In einer bevorzugten Ausführungsform wird der verdampfbare Teil der dritten Reaktionsmischung, insbesondere der Veresterung mit Methanol, welche in der dritten Reaktionsstufe erhalten wird, in gasförmiger Form (Brüden) aus den Reaktoren III abgeführt und der weiteren Aufarbeitung, beispielsweise einem Destillationsschritt, zugeführt. Wenn bevorzugt eine Kaskade aus mehreren Reaktoren III, z.B. mehreren Kesseln, verwendet wird, kann in jedem Kessel der verdampfbare Teil der entstehenden Reaktionsmischung als Brüdenstrom abgeführt und in die weitere Aufarbeitung geführt werden. Bevorzugt wird lediglich der in den letzten beiden Kesseln entstehende verdampfbare Teil der Reaktionsmischung (als dritte Reaktionsmischung) als Brüdenstrom abgeführt und in die weitere Aufarbeitung geführt.

Die Aufarbeitung der dritten Reaktionsmischungen umfasst beispielsweise geeignete Sequenz von Trennoperationen, bevorzugt ausgewählt aus Rektifikations-, Extraktions-, Strippungs- und/oder Phasentrennschritte. Bevorzugt können Verfahrensströme in die vorgeschalteten Prozessschritte rückgeführt werden, die noch zum Zielprodukt umwandelbare Zwischenprodukte wie HIBSM oder HIBS enthalten. Zudem sollte bevorzugt durch die geeignete Wahl der Betriebsparameter der Trennoperationen sichergestellt werden, dass bereits gebildetes Methylmethacrylat nicht in wertmindernder Weise in Nebenprodukte zerfällt.

Die Trennung sowie die Rückführung dieser Zwischenprodukte sind mit einem Energieaufwand verbunden, der dadurch minimiert werden kann, dass die vorgelagerten Prozessschritte möglichst geringe Anteile dieser Zwischenprodukte erzeugen. Somit wirkt sich in der Regel eine möglichst vollständige Umwandlung insbesondere von HIBS zu MAS in der Konvertierung (Verfahrensschritt (b)) vorteilhaft auf den gesamten Produktionsprozess aus.

Vorteilhaft können ein oder mehrere Stabilisatoren in verschiedenen Stoffströmen des erfindungsgemäßen Verfahrens zugesetzt werden, um eine Polymerisation des Methylmethacrylat und/oder der Methacrylsäure zu verhindern oder zu reduzieren. Beispielsweise kann ein Stabilisator zu der dritten Reaktionsmischung, erhalten nach der Veresterung, zugegeben werden. Bevorzugt wird im Amidierungs- und Konvertierungsteil Phenothiazin und andere adäquat wirkende Stabilisatoren eingesetzt; im Veresterungs- bzw. Aufarbeitungsteil werden phenolische Verbindungen, Chinone und Katechole eingesetzt. Daneben kommen Amin-N-Oxide, wie z.B. TEMPOL bzw. Kombinationen der genannten Stabilisatorsysteme zum Einsatz.

Aus der dritten Reaktionsstufe (Veresterung oder Hydrolyse) wird bevorzugt ein Abfallstrom, bestehend im Wesentlichen aus verdünnter Schwefelsäure, abgeführt. Dieser Abfallstrom wird typischerweise aus dem Verfahren ausgeschleust. Bevorzugt wird dieser Abfallstrom, insbesondere zusammen mit einem oder mehreren wässrigen Abfallströmen des erfindungsgemäßen Verfahrens, einem Verfahren zur Regenerierung von Schwefelsäure oder einem Verfahren zur Gewinnung von Ammoniumsulfat zugeführt.

In einer bevorzugten Ausführungsform umfasst das erfindungsgemäße Verfahren in der dritten Reaktionsstufe (Verfahrensschritt (c)) die Veresterung von MASA zu MMA. Daher ist eine möglichst vollständige Umwandlung von HIBA und SIBA zu MASA entscheidend für einen hohe Ausbeute des gesamten Verfahrens. Die Effizienz des erfindungsgemäßen Verfahrens kann somit ermittelt werden als Differenz der Ausbeute nach dem Verfahrensschritt der Amidierung und der Ausbeute nach dem Verfahrensschritt der Konvertierung. Die Ausbeute nach dem Verfahrensschritt der Amidierung ergibt sich aus den gemessenen Mengen an HIBA, SIBA, MAS und MASA. Die Ausbeute nach der thermischen Umwandlung ergibt sich aus den gemessenen Mengen an MAS und MASA.

Durch das erfindungsgemäße Verfahren, insbesondere die beschriebene strömungs-optimierte Konstruktion des im Konvertierungsschritt verwendeten Reaktors (insbesondere Wärmeumwandlungsapparatur), kann die auf eingesetztes ACH bezogene Gesamtausbeute an MMA und/oder MAS wesentlich verbessert werden.

Typischerweise kann die Amidierungsausbeute Werte bis zu 97 % bezüglich des eingesetzten Ausgangsprodukts ACH erreichen. Bevorzugt können sich die Ausbeuten der Konvertierungsreaktion relativ zum Stand der Technik um 0,4 bis 1% verbessern, bezogen auf die Umwandlung der nach Amidierung vorhandenen Zwischenprodukte sowie des bereits in der Amidierung vorgebildeten MASA.

Die ökonomische Trageweite der beschriebenen Erfindung verdeutlicht sich an typischen Produktionsmengen ACH-basierter MMA-Herstellungsverfahren. Für eine typische MMA-Produktionsanlage mit einer Kapazität von 250 kt/a ergibt sich durch die beschriebene Verfahrensverbesserung eine Produktions-Mehrmenge von 1000 bis 2500 t pro Jahr.

### Apparat zur Durchführung des erfindungsgemäßen Verfahrens

Die vorliegende Erfindung betrifft zudem einen Apparat zur Durchführung des erfindungsgemäßen Verfahrens wie oben beschrieben, wobei der Apparat jeweils einen oder mehrere Reaktoren I (Amidierungsreaktor), II (Konvertierungsreaktor) und III (Veresterungs/Hydrolyse-Reaktor) aufweist, welche miteinander durch Rohrleitungselemente verbunden sind, und wobei mindestens ein Reaktor II mindestens ein Vorheizer-Segment und mindestens ein Verweilzeit-Segment umfasst, wobei das Vorheizer-Segment ein oder mehreren lineare Rohrleitungselemente umfasst, die mittels eines räumlich von der Reaktionsmischung getrennten Heizmediums aufgeheizt werden, und wobei das Verweilzeit-Segment annähernd adiabatisch betrieben wird, und wobei das Vorheizer-Segment und/oder das Verweilzeit-Segment in einer Kombination aus linearen Rohrleitungselementen und Umlenkungen realisiert sind, wobei die linearen Rohrleitungselementen und die Umlenkungen über Reduzierflansche miteinander verbunden sind.

Die bevorzugten Ausführungsformen, die oben im Zusammenhang mit dem erfindungsgemäßen Verfahren beschrieben sind, gelten in analoger Weise auch für den Apparat.

Bevorzugt entspricht das Leervolumen der linearen Rohrleitungselemente relativ zum Leervolumen der Umlenkungen in mindestens einem Vorheizer-Segment einem Verhältnis im Bereich von 2,0 bis 75,0; bevorzugt im Bereich von 4,0 bis 75,0; insbesondere bevorzugt im Bereich von 5,0 bis 50,0; weiterhin bevorzugt im Bereich von 5,0 bis 20,0.

Bevorzugt werden im Vorheizer-Segment die linearen Rohrleitungselemente in ein oder mehreren geraden Rohrleitungen ausgeführt, die einen inneren Durchmesser im Bereich von 8 mm bis 200 mm, bevorzugt 12 mm bis 160 mm, aufweisen, wobei die Rohrleitungen der linearen Rohrleitungselemente im Vorheizer-Segment einen kleineren inneren Durchmesser aufweisen als die Rohrleitungen der linearen Rohrleitungselemente im Verweilzeit-Segment.

Bevorzugt werden die Umlenkungen aus Rohrleitungselementen gebildet, die eine Biegung von 90° bis 180°aufweisen.

In einer bevorzugten Ausführungsform sind die linearen Rohrleitungselemente des Vorheizer-Segments und/oder des Verweilzeit-Segments durch Umlenkungen verbunden, wobei die Umlenkungen ein um 180° umlenkendes Element darstellen, das gebildet wird aus zwei 90 °-Rohrleitungsumlenkungen und einem linearen Rohrleitungselement, das bevorzugt zwischen den 90 °-Rohrleitungsumlenkungen angeordnet ist.

Die Erfindung und insbesondere die Wirkung der strömungsoptimierten Konstruktion der für den Verfahrensschritt der Konvertierung verwendeten Wärmeumwandlungsapparatur wird anhand der folgenden Beispiele veranschaulicht.

### Beschreibung der Figuren

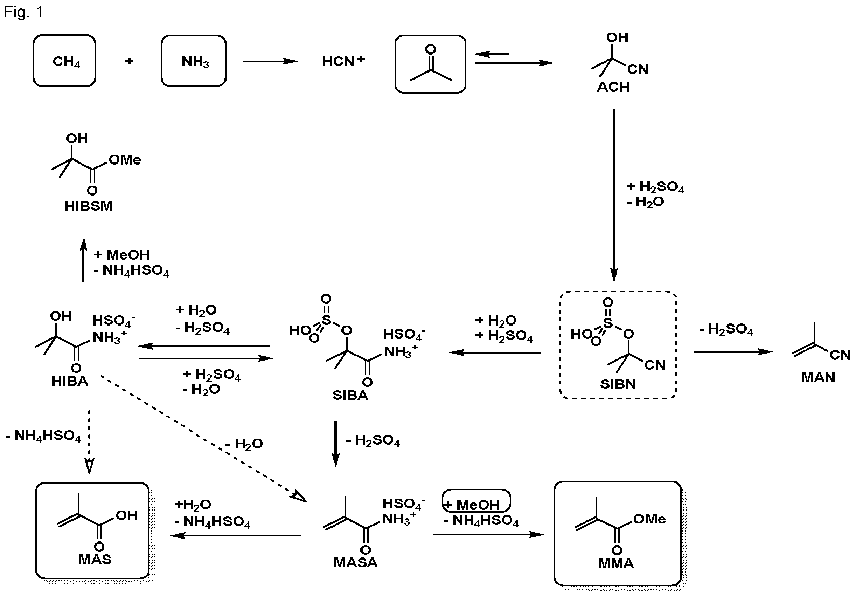

Figur 1 beschreibt das Reaktionsnetzwerk der Bildung von Methacrylsäure und/oder Methylmethacrylat ausgehend von Methan und Ammoniak sowie Aceton. Ausgehend von Methan (CH₄) und Ammoniak (NH₃) kann über das BMA-Verfahren (Blausäure aus Methan und Ammoniak) mittels katalytischer Dehydrierung Blausäure hergestellt werden CH₄ + NH₃ → HCN + 3 H₂) (Variante 1). Alternativ kann über das Andrussow-Verfahren ausgehend von Methan und Ammoniak, unter Zugabe von Sauerstoff, Blausäure hergestellt werden (CH₄ + NH₃ + 1,5 O₂ → HCN + 3 H₂O) (Variante 2). Weitere dem Fachmann bekannte Quellen für Blausäure für die Herstellung von Acetoncyanhydrin sind Acrylnitril-Prozesse, bei deren Propylen-Ammonoxidation auch 5-15 % Blausäure entstehen, relativ zum Hauptprodukt Acrylnitril, sowie das sogenannte Formamidverfahren, wobei Formamid in der Gasphase unter Dehydratisierung zu HCN umgesetzt wird.

Im nächsten Schritt wird ausgehend von Aceton und Blausäure, unter Zugabe eines basischen Katalysators (z.B. Diethylamin Et₂NH oder Alkalihydroxide) Acetoncyanhydrin (ACH) hergestellt. Die Hydroxygruppe des Acetoncyanhydrins wird im Folgenden mit Schwefelsäure verestert, wobei zunächst Sulfoxyisobutyronitril (SIBN) erhalten wird. Die Nitrilgruppe des Sulfoxyisobutyronitrils (SIBN) kann im nächsten Schritt unter Einwirkung von Schwefelsäure und Wasser verseift werden, wobei Sulfoxyisobuttersäureamid-Hydrogensulfat (SIBA·H₂SO₄) erhalten wird. Als Nebenreaktion kann die Bildung von Methacrylnitril (MAN) unter Eliminierung von Schwefelsäure aus SIBN erfolgen. Sulfoxyisobuttersäureamid-Hydrogensulfat (SIBA·H₂SO₄) kann zudem teilweise zum alpha-Hydroxyisobuttersäureamid-hydrogensulfat (alpha-Hydroxyisobutyramidhydrogensulfat, HIBA·H₂SO₄) hydrolysiert werden. Ebenfalls ist die Rückreaktion zum Schwefelsäureester SIBA·H₂SO₄ möglich. Als Nebenprodukt kann alpha-Hydroxyisobuttersäure (HIBS) durch weitere Hydrolyse von HIBA·H₂SO₄ gebildet werden. Ausgehend von SIBA·H₂SO₄ wird unter Eliminierung von Schwefelsäure Methacrylamidhydrogensulfat (MASA·H₂SO₄)gebildet (Konvertierung). Ebenfalls kann die langsame Umsetzung von HIBA oder HIBS zu MAS oder MASA als Eliminierungsreaktion unter Abspaltung von NH₄HSO₄ oder Wasser ablaufen. Nachfolgend kann Methacrylamidhydrogensulfat (MASA·H₂SO₄) durch Hydrolyse zu Methacrylsäure (MAS) oder durch Veresterung mit Methanol (MeOH) zu Methylmethacrylat (MMA) umgewandelt werden. Wird alpha-Hydroxyisobuttersäure (HIBS) in die Veresterung mitgeschleppt, kann es zu alpha-Hydroxyisobuttersäuremethylester (HIBSM) umgesetzt werden; zum gleichen Reaktionsprodukt führt die Umsetzung von HIBA mit Methanol.

Die Abkürzungen in Figur 1 und der Beschreibung zu Figur 1 haben die folgenden Bedeutungen:
- ACH: Acetoncyanhydrin;
- SIBN: alpha-Sulfoxy-isobuttersäurenitril, auch als Sulfoxyisobutyronitril bezeichnet;
- SIBA: alpha-Sulfoxyisobuttersäureamid, auch als Sulfoxyisobuttersäureamid oder Sulfoxyisobutyramid bezeichnet;
- SIBA·H₂SO₄: alpha-Sulfoxyisobuttersäureamid-Hydrogensulfat, auch als Sulfoxyisobuttersäureamid-Hydrogensulfat bezeichnet;
- MAN: Methacrylnitril;
- HIBA: alpha-Hydroxyisobuttersäureamid; alpha-Hydroxyisobutyramid;
- HIBA·H₂SO₄: alpha-Hydroxyisobuttersäureamid-Hydrogensulfat, alpha-Hydroxyisobutyramid-Hydrogensulfat;
- MASA: Methacrylsäureamid / Methacrylamid;
- MASA·H₂SO₄: Methacrylsäureamid-Hydrogensulfat;
- MAS: Methacrylsäure;
- MMA: Methylmethacrylat;
- HIBS: alpha-Hydroxyisobuttersäure;
- HIBSM: alpha-Hydroxyisobuttersäuremethylester.

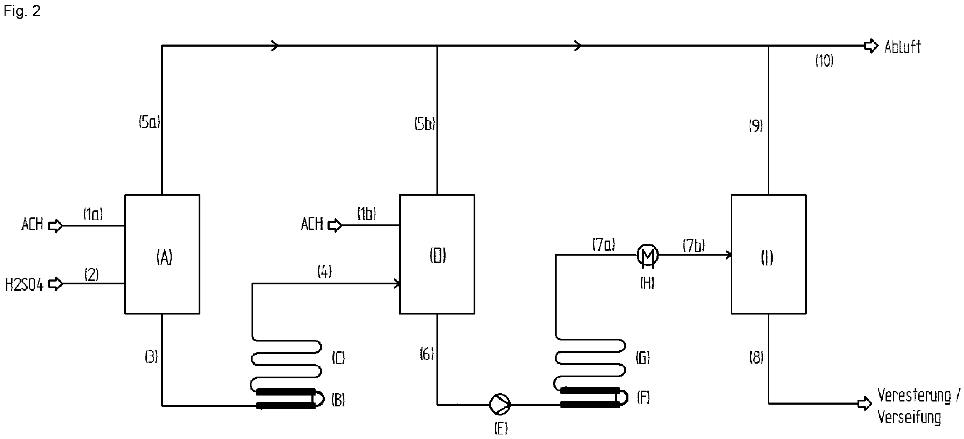

Figur 2 zeigt ein Fließschema einer bevorzugten Ausführungsform der ersten und zweiten Reaktionsstufen (Amidierung und Konvertierung) des erfindungsgemäßen Verfahrens. In Figur 1 sind zwei aufeinander folgende Verfahrensschritte der Amidierung und Konvertierung umfassend die erste Amidierung (A) und die erste Konvertierung ((B), (C)) sowie die zweite Amidierung (D) und die zweite Konvertierung ((F), (G)).

In Figur 2 haben die Bezugszeichen die folgenden Bedeutungen:
Apparate:
   (A) Amidierungsreaktor 1
   (B) Vorheizer-Segment 1
   (C) Verweilzeit-Segment 1
   (D) Amidierungsreaktor 2
   (E) Austragspumpe
   (F) Vorheizer-Segment 2
   (G) Verweilzeit-Segment 2
   (H) Kühler
   (I) Pufferbehälter
Stoffströme:
   (1a) Acetoncyanhydrin-Zuführung zu Reaktor 1
   (1b) Acetoncyanhydrin-Zuführung zu Reaktor 2
   (2) Schwefelsäure-Zuführung zu Reaktor 1
   (3) Austrag Amidierungsreaktor 1 (flüssig)
   (4) Reaktionsgemisch nach Zwischenkonvertierung
   (5a) Abgas Amidierungsreaktor 1
   (5b) Abgas Amidierungsreaktor 2
   (6) Austrag Amidierungsreaktor 2 (flüssig)
   (7a) Reaktionsgemisch nach Konvertierung
   (7b) Gekühltes Reaktionsgemisch
   (8) Austrag Pufferbehälter (flüssig)
   (9) Abgas aus Pufferbehälter
   (10) Gesamtabgas

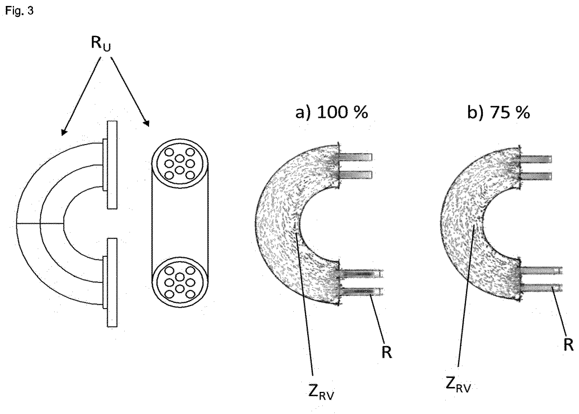

Figur 3 zeigt eine 180° Rohrumlenkung Ru im Vorheizer-Segment (B) und/oder (F) gemäß der Beispiel 1 und 2 sowie die Darstellung der Strömungsverhältnisse darin. Unter a) sind die Strömungsverhältnisse bei 100 % Last und unter b) die Strömungsverhältnisse bei 75 % Last dargestellt. Es führen jeweils acht lineare Rohrleitungen R in die Rohrumlenkung Ru. Zonen starker Rückvermischung in der Rohrumlenkung sind durch Z_{RV} markiert.

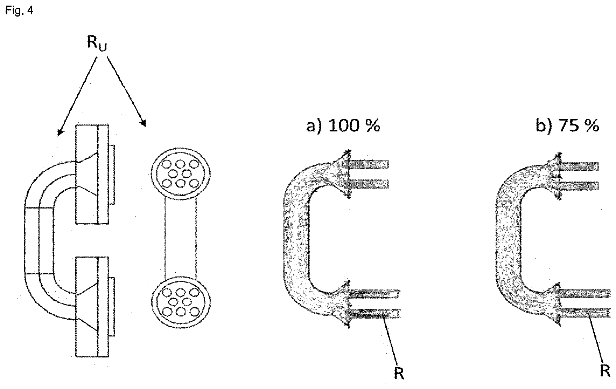

Figur 4 zeigt eine 180° Rohrumlenkung Ru im Vorheizer-Segment (B) und/oder (F) gemäß den erfindungsgemäßen Beispielen 3 bis 6 sowie die Darstellung der Strömungsverhältnisse hierin bei a) 100 % Last (siehe Beispiele 3, 4, 5) und b) 75 % Last (siehe Beispiel 6). Es führen jeweils acht lineare Rohrleitungen R in die Rohrumlenkung Ru.

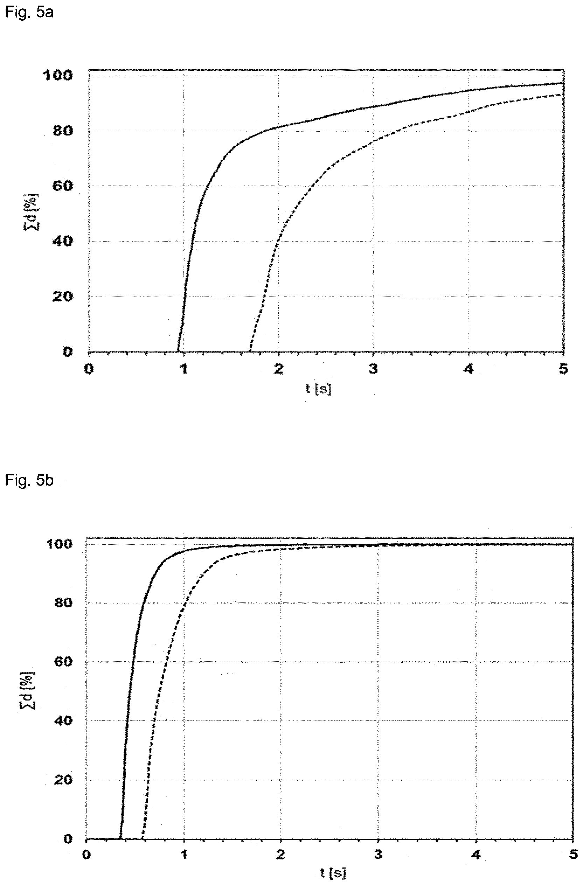

Figur 5a zeigt die Verweilzeitverteilung (Summenfunktion) in einer Rohrumlenkung gemäß Vergleichsbeispielen gemäß Figur 3 bei 100 % Last (Durchfluss) in der durchgezogenen Linie und 75 % Last (Durchfluss) in der gestrichelten Linie. Figur 5b zeigt die Verweilzeitverteilung (Summenfunktion) in einer Rohrumlenkung gemäß erfindungsgemäßen Beispielen gemäß Figur 4 bei 100 % Last (Durchfluss) in der durchgezogenen Linie und 75 % Last (Durchfluss) in der gestrichelten Linie. Hierin bedeuten:
Σd: Summenverteilung (Integral der Anzahldichteverteilung von 12.000 betrachteten Volumenelementen);
τ: Verweilzeit

Die Erfindung wird anhand der folgenden Beispiele weiter beschrieben.

### Beispiele

Die Herstellung von Methacrylamid in schwefelsaurer Lösung, umfassend die Umsetzung von Acetoncyanhydrin mit Schwefelsäure in der Amidierung, der thermischen Umsetzung des Gemischs in der Konvertierung sowie dessen anschließender Abkühlung, erfolgte anhand der Ausführungsform nach Figur 2.

Im Folgenden werden zwei Vergleichsbeispiele (Beispiel 1 sowie Beispiel 2) unter Verwendung einer Konstruktion der Erhitzung / Konvertierung gemäß dem Stand der Technik (nach Figur 3) sowie erfindungsgemäße Beispiele (Beispiele 3-6) unter Verwendung einer strömungs-optimierten Konstruktion (nach Figur 4) der für den Verfahrensschritt der Konvertierung verwendeten Wärmeumwandlungsapparatur beschrieben.

Dabei wurden Proben nach dem Konvertierungs-Reaktor entnommen. Die nach Quantifizierung mittels HPLC ermittelten Konzentrationen von MASA, MAS und HIBA wurden für die Bilanzierung der Verfahrensschritte der Amidierung und der Konvertierung herangezogen. Ergebnisse und Gesamtausbeuteverluste über die einzelnen Verfahrensschritte sind für die Vergleichsbeispiele 1 und 2 und die erfindungsgemäßen Beispiele 3-6 in den Tabellen 1-7 dargestellt. Bei Auflistung der Messergebnisse ist jeweils ein Messfehler der HPLC-Analytik von ± 0.2 % ausgewiesen.

Das aus der Konvertierung erhaltene schwefelsaure Gemisch, enthaltend MASA, MAS und HIBA kann anschließend mit Methanol und Wasser zu Methylmethacrylat umgesetzt werden oder mit Wasser zur Methacrylsäure hydrolysiert werden.

### Beispiel 1 (Vergleichsbeispiel): Herstellung von Methacrylamid in schwefelsaurer Lösung auf Basis von Acetoncyanhydrin

Die allgemeine Durchführung des Verfahrens gemäß Fließschema nach Figur 2 ist im Folgenden beschrieben. Im Beispiel 1 (Vergleichsbeispiel) wurden Rohrumlenkungen gemäß dem Stand der Technik wie in Figur 3 dargestellt verwendet. Die Ergebnisse, repräsentativ für 7 Tage stationären Betriebes, sind in Tabelle 1 dargestellt.

11000 kg/h Acetoncyanhydrin mit einer typischen Zusammensetzung von 99,0 % Acetoncyanhydrin; 0,3 % Aceton; 0,4 % Wasser sowie Schwefelsäure wurde im Massenverhältnis von 65/35 aufgeteilt, so dass ein Strom (1a) mit 7100 kg/h und ein Strom (1b) mit 3900 kg/h erhalten wurden.

Der Wassergehalt des ACH-Zuleitungsstroms (1a) und (1b) berechnet sich aus der Differenz zum ACH-Gehalt, der mittels HPLC ermittelt wird.

Der Wassergehalt in der Schwefelsäure-Zuleitung (2) berechnet sich aus der Differenz zum Schwefelsäuregehalt, der durch Messung der Dichte und Schallgeschwindigkeit ermittelt wird.

Zulaufstrom (1a) wurde anschließend auf den ersten Amidierungsreaktor (A) aufgegeben. Die Schwefelsäure (2) wurde vor Eintritt in den Amidierungsreaktor mit 50 ppm Phenothiazin (als Beispiel eines Stabilisators gem. Stand der Technik) versetzt.

Der Amidierungsreaktor (A) war als Schlaufenreaktor ausgeführt und wurde bei 100°C betrieben. Der Zulaufstrom (1a) wurde kontinuierlich und mit einer Temperatur von 20°C dem beschriebenen Reaktorkreislauf (A) zugeführt.

Die für die optimale Umsetzung des Reaktionsgemischs in den Reaktoren (A) und (D) notwendige Schwefelsäuremenge (2), welche eine Konzentration von 99,7 % (0,3 % Wasser) aufweist, wurde in einem Masseverhältnis zur Gesamtmenge Acetoncyanhydrin (1a+1b) von 1,62 kgH₂SO₄/kgACH Reaktor (A) zugeführt. Der eingesetzte Zulaufmassestrom an Schwefelsäure belief sich auf 17800 kg/h. Hierdurch wurde in Reaktor (A) ein Schwefelsäureüberschuss (Schwefelsäure/ACH Verhältnis) von 2,55 kg/kg realisiert.

Das erhaltene 100°C heiße Anrührgemisch (3), bestehend aus Sulfoxyisobuttersäureamid (SIBA), Methacrylsäureamid (MASA) sowie Hydroxyisobuttersäureamid (HIBA), gelöst in Schwefelsäure, wurde bei gleichzeitig erfolgter Gasabscheidung im Kreislaufbetrieb kontinuierlich einer Zwischenkonvertierung (B, C) zugeführt. Die zur Förderung benötigte Druckdifferenz wurde durch die Reaktorkreislaufpumpe des Amidierungsreaktors (A) realisiert. Das entstehende Abgas (5a) wurde in Richtung Abluft (10) aus dem Prozess abgeführt.

Der erste Konvertierungsreaktor (B, C) war als Strömungsrohrreaktor ausgeführt, bestehend aus einem Vorheizer-Segment (B) und einem Verweilzeit-Segment (C). Das in den Zwischenkonvertierungsreaktor (B, C) eintretende Reaktionsgemisch wurde zunächst auf zwei Teilströme aufgeteilt und im Vorheizer-Segment (B), bestehend aus jeweils 3 seriell verschalteten Rohrbündelwärmeüberträgern der Nennweite DN150, auf 130°C am prozessseitigen Austritt der Apparatur erhitzt.

Das Reaktionsmedium wurde im Vorheizer-Segment (B) ausschließlich auf der Rohrseite geführt, während der jeweilige Wärmetauscher-Mantel mit 12 bar(g) Sattdampf beheizt war. Jeder Wärmetauscher war mit 8 Rohren, welche einen Innendurchmesser von 18 mm aufwiesen, versehen, so dass eine Strömungsgeschwindigkeit in den Rohren von 1,22 m/s erhalten wurde.

Die einzelnen Wärmeüberträger wurden als Verbund in einem Gestell parallel zueinander angeordnet, wobei die prozessseitige Verbindung der seriell verschalteten Apparate mittels zweier je um 90° gebogenen Rohrleitungssegmente (zusammen 180°) realisiert war. Im Vorheizer-Segment (B) wurde weiterhin ein Leervolumen-Verhältnis der linearen Rohrleitungsabschnitte relativ zu den gebogenen Rohrleitungsabschnitten bzw. relativ zu den Umlenkungen V_{Linear}/V_{Umlenkung} gemäß Tabelle 7 realisiert.

Die Nennweite der verbindenden Rohrleitungselemente entsprach jener des Mantels der Rohrbündelapparate und belief sich ebenfalls auf DN150. (Innendurchmesser di = 158 mm). Hierdurch wird eine mittlere Strömungsgeschwindigkeit des Reaktionsmediums, gemäß Tabelle 7, in diesen Abschnitten erhalten.

Das auf diese Weise erhitzte Reaktionsgemisch wurde anschließend aus den beiden parallel geführten Vorheizer-Segmenten (B) zusammengeführt und durch ein annähernd adiabatisch betriebenes Verweilzeit-Segment (C) geleitet. Das Verweilzeitsegment (C) setzte sich aus 12 seriell verschalteten, linearen Reaktorsegmenten zusammen, welche, wie in den Vorheizer-Segmenten, parallel zueinander angeordnet und mit 180° Rohrbögen der gleichen Nennweite miteinander verbunden waren.

Die Rohre des Verweilzeitsegments wie auch die gebogenen verbindenden Rohrleitungselemente sind mit einem Innendurchmesser von di = 158 mm ausgeführt und bedingen damit eine Strömungsgeschwindigkeit des Reaktionsgemischs, wie in Tabelle 7 dargestellt. Im Verweilzeit-Segment (C) wurde ein Leervolumen-Verhältnis der linearen Rohrleitungsabschnitte relativ zu den gebogenen Rohrleitungsabschnitten bzw. relativ zu den Umlenkungen V_{Linear}/V_{Umlenkung} gemäß Tabelle 7 realisiert. Gegeben durch das Volumen des Erhitzungssegments (B) des Verweilzeit-Segments (C) sowie der verbindenden Rohrleitungen wurde in der Zwischenkonvertierung (B, C) eine mittlere statische Verweilzeit gemäß Tabelle 7 erreicht.

Das aus dem ersten Konvertierungsreaktor (B, C) austretende Reaktionsgemisch (4) wurde im Anschluss dem zweiten Amidierungsreaktor (D) zugeführt. Der Amidierungsreaktor (D) ist analog zu Reaktor (A) als Schlaufenreaktor aufgebaut und wurde ebenfalls bei ca. 100°C betrieben. Die für den zweiten Reaktionsschritt notwendige Menge von 3900 kg/h Acetoncyanhydrin (1b) wurde auch im zweiten Amidierungsreaktor (D) direkt in die Reaktionsmischung aufgegeben. Das sich in der Reaktionsmischung einstellende Masseverhältnis ergab sich hierdurch zu 1,62 kg H₂SO₄/kg ACH. Das entstehende Abgas (5b) wurde in Richtung Gesamtabgas (10) aus dem Prozess abgeführt.

Dem zweiten Amidierungsreaktor (D) wurde mittels einer Austragspumpe (E) volumenstromgeregelt und kontinuierlich ein 100°C heißes Reaktionsgemisch (6), bestehend aus Sulfoxyisobuttersäureamid (SIBA), Methacrylamid (MASA) sowie Hydroxyisobuttersäureamid (HIBA) (gelöst in Schwefelsäure), entzogen, welches zur finalen Umsetzung der reaktiven Bestandteile dem zweiten Konvertierungsschritt (F, G) zugeführt wurde.

Der zweite Konvertierungsreaktor (F, G) war ebenfalls als Strömungsrohrreaktor ausgeführt, bestehend aus einem Vorheizer-Segment (F) und einem Verweilzeit-Segment (G). Das in den Konvertierungsreaktor (F, G) eintretende Reaktionsgemisch wurde zunächst auf zwei Teilströme aufgeteilt und anschließend im Erhitzungssegment (F), bestehend aus jeweils 12 seriell verschalteten Rohrbündelwärmeüberträgern der Nennweite DN150, auf 158°C am prozessseitigen Austritt der Apparatur erhitzt. Das Reaktionsmedium wurde im Vorheizer-Segment (F) ausschließlich auf der Rohrseite geführt, während der jeweilige Wärmetauscher-Mantel mit 12 bar(g) Stattdampf beheizt war.

Jeder Wärmetauscher war mit 8 Rohren, welche einen Innendurchmesser von 18 mm aufweisen, versehen, so dass eine Strömungsgeschwindigkeit in den Rohren gemäß Tabelle 7 erhalten wurde. Die einzelnen Wärmeübertrager waren als Verbund in einem Gestell parallel zueinander angeordnet, wobei die prozessseitige Verbindung der seriell verschalteten Apparate mittels zweier je um 90° gebogenen Rohrleitungssegmente (zusammen 180°) realisiert war. Im Vorheizer-Segment (F) wurde weiterhin ein Leervolumen-Verhältnis der linearen Rohrleitungsabschnitte relativ zu den gebogenen Rohrleitungsabschnitten bzw. relativ zu den Umlenkungen V_{Linear}/V_{Umlankung} gemäß Tabelle 7 realisiert. Die Nennweite der verbindenden Rohrleitungselemente entsprach jener des Mantels der Rohrbündelapparate und belief sich ebenfalls auf DN150. Hierdurch wurde eine Strömungsgeschwindigkeit gemäß Tabelle 7 in diesen Abschnitten erhalten.

Nach erfolgreicher Vereinigung der beiden 158°C heißen Reaktionsgemische wurde die resultierende Mischung anschließend durch ein adiabat betriebenes Verweilzeit-Segment (G) geleitet. Das Verweilzeit-Segment (G) setzte sich aus 13 seriell verschalteten, linearen Reaktorsegmenten zusammen, welche in analoger Weise wie die Vorwärmer parallel zueinander angeordnet und mit 180° Rohrbögen derselben Nennweite miteinander verbunden waren.

Die Reaktoren des Verweilzeit-Segments (G) waren ebenso wie auch die verbindenden Rohrleitungselemente mit einem Innendurchmesser von dᵢ = 158 mm ausgeführt und bedingen damit eine Strömungsgeschwindigkeit des Reaktionsgemischs gemäß Tabelle 7. Im Verweilzeit-Segment (G) wurde weiterhin ein Leervolumen-Verhältnis der linearen Rohrleitungsabschnitte relativ zu den gebogenen Rohrleitungsabschnitten bzw. relativ zu den Umlenkungen V_{Linear}/V_{Umlenkung} gemäß Tabelle 7 realisiert. Gegeben durch die Leerrohr-Volumina des Vorheizer-Segments (F), des Verweilzeit-Segments (G) sowie der verbindenden Rohrleitungen wurde im zweiten Konvertierungsreaktor (F, G) eine mittlere statische Verweilzeit gemäß Tabelle 7 erreicht.

Nach der zweiten Konvertierung (F, G), wurde ein heißes, mit Methacrylsäureamid (MASA) angereichertes, Reaktionsgemischs (7a) erhalten, welches anschließend in einem mit Warmwasser betriebenem Kühler auf 100°C abgekühlt und anschließend als Strom (7b) einem Pufferbehälter (I) zugeführt wurde. Die mittlere Verweilzeit des abgekühlten Reaktionsgemischs (7b) in Pufferbehälter (I) ist in Tabelle 7 ausgewiesen.

Im Zwischenbehälter (I) wurde das in der Reaktionsmatrix freiwerdende Gas (9) abgetrennt und mit den Abgasen (5a) und (5b) zum Gesamtabgas (10) zusammengeführt. Es wurde so ein Gesamtabgas (10) von ca. 95 m³/h erhalten, welches kontinuierlich aus dem Prozess entfernt wurde.

Aus dem im Zwischenbehälter (I) gesammelten Reaktionsgemisch wurde kontinuierlich ein flüssiger Produktstrom (8) von ca. 28700 kg/h entzogen, welcher mittels HPLC hinsichtlich der Komponenten Methacrylsäureamid (MASA), Methacrylsäure (MAS) sowie Hydroxyisobuttersäureamid (HIBA) analysiert wurde. Die jeweilige Analyse wurde dreifach durchgeführt; die jeweiligen arithmetischen Mittelwerte sind in Tabelle 1 eingetragen. Probenahme und Analyse erfolgten zweimal pro Tag, wobei im stationären Betrieb der Anlage insgesamt 7 Tage in Folge Proben erhoben wurden.

Die erhaltene Summenausbeute zum Zielprodukt umsetzbarer Komponenten (Methacrylsäureamid, Methacrylsäure), welche anschließend einer Veresterung zur Herstellung von Methylmethacrylat oder einer Hydrolyse zur Herstellung von Methacrylsäure zugeführt wurde, ist ebenfalls in Tabelle 1 dargestellt. Die mittlere Ausbeute von 14 repräsentativen Proben, gemessen im stationären Betrieb eines Prozesses mit den genannten Bedingungen, betrug 94,1%.

**Tabelle 1: Ergebnisse gemäß Vergleichsbeispiel 1**

| | | Konzentration im Produktstrom [%] | | | Ausbeute [%] |
|---|---|---|---|---|---|
| Probe | Zeit | MASA | MAS | HIBA | MASA + MAS |
| 1 | Tag 1; 10:00 | 35,76 | 1,06 | 0,17 | 94,26 |
| 2 | Tag 1; 13:50 | 35,81 | 1,04 | 0,17 | 94,26 |
| 3 | Tag 2; 07:40 | 35,75 | 1,03 | 0,15 | 94,09 |
| 4 | Tag 2; 11:23 | 35,67 | 1,06 | 0,16 | 93,95 |
| 5 | Tag 3; 08:05 | 35,68 | 1,05 | 0,17 | 94,07 |
| 6 | Tag 3; 11:05 | 35,71 | 1,06 | 0,16 | 93,95 |
| 7 | Tag 4; 09:34 | 35,89 | 1,04 | 0,16 | 94,51 |
| 8 | Tag 4; 13:10 | 35,86 | 1,03 | 0,17 | 94,52 |
| 9 | Tag 5; 08:40 | 35,79 | 1,05 | 0,16 | 94,28 |
| 10 | Tag 5; 11:18 | 35,83 | 1,05 | 0,16 | 94,35 |
| 11 | Tag 6; 09:45 | 35,72 | 1,02 | 0,16 | 93,84 |
| 12 | Tag 6; 13:00 | 35,86 | 1,04 | 0,16 | 94,51 |
| 13 | Tag 7; 08:40 | 35,58 | 1,07 | 0,10 | 93,84 |
| 14 | Tag 7; 12:40 | 35,77 | 1,01 | 0,15 | 94,22 |
| Mittlere Ausbeute bez. (MASA+MAS) | | | | | ∅ 94,1 |
| Schwankungsbreite der Mittleren Ausbeute | | | | | ± 0,59 % |

Beispiel 2 (Vergleichsbeispiel): Prozess zur Herstellung von Methacrylamid in schwefelsaurer Lösung auf Basis von Acetoncyanhydrin unter Verwendung von Oleum 100,6%

Die allgemeine Durchführung des Verfahrens mit Konstruktion des Konvertierungsreaktors gemäß Stand der Technik sowie unter Einsatz von Oleum (100,6 %) ist im Folgenden beschrieben, wobei die die jeweiligen Prozessbedingungen in Tabelle 2 bzw. 7 detailliert dargestellt sind. Soweit nicht anders angegeben, wurde das Beispiel unter identischen Prozessbedingungen wie im Beispiel 1 durchgeführt.

Im Beispiel 2 (Vergleichsbeispiel) wurden Rohrumlenkungen wie in Figur 3 (Stand der Technik) dargestellt verwendet. Die Ergebnisse, repräsentativ für 7 Tage stationären Betriebes, sind in Tabelle 2 dargestellt.

Am Pufferbehälter (I) wurde ein Stoffstrom (8) erhalten, welcher eine Konzentration von Methacrylamid (MASA), Methacrylsäure (MAS) sowie Hydroxy-isobuttersäureamid (HIBA) gemäß Tabelle 2 aufwies.

Die mittlere Ausbeute von 14 repräsentativen Proben, gemessen im stationären Betrieb des Prozesses mittels HPLC, betrug unter den genannten Bedingungen 90,9%.

**Tabelle 2: Ergebnisse gemäß Beispiel 2**

| | | Konzentration im Produktstrom [%] | | | Ausbeute [%] |
|---|---|---|---|---|---|
| Probe | Zeit | MASA | MAS | HIBA | MASA + MAS |
| 1 | Tag 1; 07:22 | 34,75 | 0,14 | 0,02 | 91,60 |
| 2 | Tag 1; 10:56 | 34,50 | 0,15 | 0,02 | 90,94 |
| 3 | Tag 2; 08:07 | 34,63 | 0,14 | 0,02 | 91,28 |
| 4 | Tag 2; 10:05 | 34,54 | 0,13 | 0,06 | 91,05 |
| 5 | Tag 3; 07:03 | 34,22 | 0,10 | 0,04 | 90,21 |
| 6 | Tag 3; 11:16 | 34,68 | 0,10 | 0,01 | 91,42 |
| 7 | Tag 4; 09:49 | 34,67 | 0,13 | 0,10 | 91,39 |
| 8 | Tag 4; 12:10 | 34,58 | 0,15 | 0,02 | 91,15 |
| 9 | Tag 5; 08:41 | 34,19 | 0,14 | 0,03 | 90,13 |
| 10 | Tag 5; 11:53 | 34,04 | 0,14 | 0,03 | 89,73 |
| 11 | Tag 6; 08:10 | 34,62 | 0,14 | 0,12 | 91,15 |
| 12 | Tag 6; 11:10 | 34,53 | 0,13 | 0,08 | 90,85 |
| 13 | Tag 7; 08:21 | 34,21 | 0,09 | 0,04 | 90,60 |
| 14 | Tag 7; 10:33 | 34,65 | 0,10 | 0,06 | 91,41 |
| Mittlere Ausbeute bez. (MASA+MAS) | | | | | ∅ 90,9 |
| Schwankungsbreite der Mittleren Ausbeute | | | | | 1,87 % |

Beispiel 3 (erfindungsgemäß): Prozess zur Herstellung von Methacrylamid in schwefelsaurer Lösung auf Basis von Acetoncyanhydrin mit strömungs-optimierten Konvertern.

Die allgemeine Durchführung des Verfahrens mit strömungs-optimierter Konstruktion der Konvertierungsreaktors ist im Folgenden beschrieben, wobei die die jeweiligen Prozessbedingungen in Tabelle 3 bzw. 7 dargestellt sind. Soweit nicht anders angegeben, wurde der erfindungsgemäße Prozess unter identischen Prozessbedingungen wie im Beispiel 1 durchgeführt.

Im erfindungsgemäßen Beispiel 3 wurden Rohrumlenkungen wie in Figur 4 (erfindungsgemäß) dargestellt verwendet. Die Ergebnisse, repräsentativ für 7 Tage stationären Betriebes, sind in Tabelle 3 dargestellt. Durch die erfindungsgemäßen konstruktiven Veränderungen an den Vorheizer-Segmenten (B) bzw. (F) ergaben sich in den Konvertern (B, C) sowie (F, G) neue Strömungs- und Reaktionsbedingungen, welche in Tabelle 7 zusammengefasst sind. Die jeweilige Auswirkung auf die Ausbeute des Prozesses ist weiterhin in Tabelle 3 dargestellt.

Am Pufferbehälter (I) wurde ein Stoffstrom (8) erhalten, welcher eine Konzentration von Methacrylamid (MASA), Methacrylsäure (MAS) sowie Hydroxy-isobuttersäureamid (HIBA) gemäß Tabelle 3 aufwies.

Die mittlere Ausbeute von 14 repräsentativen Proben, gemessen im stationären Betrieb des Prozesses mittels HPLC, betrug unter den genannten Bedingungen 96,5%.

**Tabelle 3: Ergebnisse gemäß Beispiel 3**

| | | Konzentration im Produktstrom [%] | | | Ausbeute [%] |
|---|---|---|---|---|---|
| Probe | Zeit | MASA | MAS | HIBA | MASA + MAS |
| 1 | Tag 1; 08:45 | 36,33 | 0,95 | 0,23 | 96,60 |
| 2 | Tag 1; 10:45 | 36,22 | 0,98 | 0,18 | 96,60 |
| 3 | Tag 2; 10:00 | 36,13 | 0,99 | 0,18 | 95,91 |
| 4 | Tag 2; 11:30 | 35,57 | 0,97 | 0,14 | 96,69 |
| 5 | Tag 3; 10:25 | 36,30 | 1,05 | 0,14 | 96,73 |
| 6 | Tag 3; 11:25 | 35,98 | 1,09 | 0,13 | 96,22 |
| 7 | Tag 4; 14:35 | 36,42 | 0,90 | 0,23 | 96,83 |
| 8 | Tag 4; 16:45 | 36,26 | 0,90 | 0,24 | 96,43 |
| 9 | Tag 5; 08:50 | 36,21 | 0,94 | 0,22 | 96,37 |
| 10 | Tag 5; 13:00 | 36,39 | 0,93 | 0,20 | 96,71 |
| 11 | Tag 6; 09:45 | 36,35 | 0,87 | 0,19 | 96,49 |
| 12 | Tag 6; 12:30 | 36,40 | 0,95 | 0,21 | 96,67 |
| 13 | Tag 7; 10:00 | 36,38 | 0,87 | 0,24 | 96,64 |
| 14 | Tag 7; 12:50 | 35,58 | 0,85 | 0,21 | 96,56 |
| Mittlere Ausbeute bez. (MASA+MAS) | | | | | ∅ 96,5 |
| Schwankungsbreite der Mittleren Ausbeute | | | | | ± 0,32 % |

Beispiel 4 (erfindungsgemäß): Prozess zur Herstellung von Methacrylamid in schwefelsaurer Lösung auf Basis von Acetoncyanhydrin mit strömungs-optimierten Konvertern und optimaler Schwefelsäurekonzentration

Die Ausbeuteergebnisse der Kombination aus optimaler Schwefelsäurekonzentration und strömungs-optimierter Konstruktion der Konvertierungsreaktoren sind in der nachfolgenden Tabelle 4 aufgeführt.

Soweit nicht anders angegeben, wurde der erfindungsgemäße Prozess unter identischen Prozessbedingungen wie im Beispiel 1 durchgeführt. Anders als in Bespiel 1 wurde hier eine Schwefelsäurekonzentration von 99,5% H2SO4 (0,5% Wasser) statt einer Schwefelsäurekonzentration von 99,7% eingesetzt.

Im erfindungsgemäßen Beispiel 4 wurden Rohrumlenkungen wie in Figur 4 (erfindungsgemäß) dargestellt verwendet. Die Ergebnisse, repräsentativ für 7 Tage stationären Betriebes, sind in Tabelle 4 dargestellt. Durch die erfindungsgemäßen konstruktiven Veränderungen an den Vorheizer-Segmenten (B) bzw. (F) ergaben sich in der Konvertierung (B, C) sowie (F, G) annähernd gleiche Strömungs- und Reaktionsbedingungen, welche in Tabelle 7 zusammengefasst sind.

Die mittlere Ausbeute von 14 repräsentativen Proben, gemessen im stationären Betrieb des Prozesses mittels HPLC, betrug unter den oben genannten Bedingungen 97,0 %.

**Tabelle 4: Ergebnisse gemäß Beispiel 4**

| | | Konzentration im Produktstrom [%] | | | Ausbeute [%] |
|---|---|---|---|---|---|
| Probe | Zeit | MASA | MAS | HIBA | MASA + MAS |
| 1 | Tag 1; 07:20 | 36,54 | 0,92 | 0,09 | 97,11 |
| 2 | Tag 1; 09:15 | 36,36 | 1,00 | 0,21 | 96,79 |
| 3 | Tag 2; 09:00 | 36,51 | 0,94 | 0,22 | 97,01 |
| 4 | Tag 2; 10:38 | 36,39 | 0,90 | 0,26 | 97,02 |
| 5 | Tag 3; 08:40 | 36,47 | 0,93 | 0,25 | 97,04 |
| 6 | Tag 3; 10:40 | 36,54 | 0,83 | 0,26 | 97,25 |
| 7 | Tag 4; 07:45 | 36,61 | 0,91 | 0,22 | 97,09 |
| 8 | Tag 4; 10:40 | 36,57 | 1,03 | 0,17 | 97,09 |
| 9 | Tag 5; 08:45 | 36,42 | 1,16 | 0,14 | 97,02 |
| 10 | Tag 5; 10:00 | 36,10 | 0,69 | 0,43 | 97,03 |
| 11 | Tag 6; 08:00 | 36,23 | 0,75 | 0,33 | 96,65 |
| 12 | Tag 6; 10:40 | 36,14 | 0,88 | 0,27 | 96,60 |
| 13 | Tag 7; 08:45 | 36,53 | 0,95 | 0,23 | 97,12 |
| 14 | Tag 7; 10:45 | 36,43 | 0,98 | 0,18 | 96,95 |
| Mittlere Ausbeute bez. (MASA+MAS) | | | | | ∅ 97,0 |
| Schwankungsbreite der Mittleren Ausbeute | | | | | ± 0,33 % |

Beispiel 5 (erfindungsgemäß): Prozess zur Herstellung von Methacrylamid in schwefelsaurer Lösung auf Basis von Acetoncyanhydrin mit strömungs-optimierten Konvertern und optimaler Schwefelsäurekonzentration bei erhöhter Verweilzeit und Temperatur des Produktgemisches im Pufferbehälter

Im erfindungsgemäßen Beispiel 5 wurden Rohrumlenkungen wie in Figur 4 (erfindungsgemäß) dargestellt verwendet. Die Ergebnisse, repräsentativ für 7 Tage stationären Betriebes, sind in Tabelle 5 dargestellt. Die Ausbeuteergebnisse der Kombination aus optimaler Schwefelsäure-konzentration, strömungs-optimierten Konvertern und erhöhter Verweilzeit der Produktgemischs im Pufferbehälter (I) ist in der nachfolgenden Tabelle 5 abgebildet.

Wenn nicht anders angegeben, wurde der erfindungsgemäße Prozess bei identischen Prozessbedingungen wie in Beispiel 4 durchgeführt. Abweichend zu den vorangegangenen Beispielen wurde das Reaktionsgemisch (7b) jedoch auf 116 °C abgekühlt, und die mittlere Verweilzeit des Gemischs (7b) im Pufferbehälter (I) betrug 51 min.

Die mittlere Ausbeute von 14 repräsentativen Proben, gemessen im stationären Betrieb des Prozesses mittels HPLC, betrug unter den genannten Bedingungen 96,4%.

**Tabelle 5: Ergebnisse gemäß Beispiel 5**

| | | Konzentration im Produktstrom [%] | | | Ausbeute [%] |
|---|---|---|---|---|---|
| Probe | Zeit | MASA | MAS | HIBA | MASA + MAS |
| 1 | Tag 1; 07:10 | 35,73 | 0,75 | 0,22 | 96,15 |
| 2 | Tag 1; 11:05 | 36,14 | 0,88 | 0,17 | 96,00 |
| 3 | Tag 2; 09:00 | 36,33 | 0,95 | 0,17 | 96,40 |
| 4 | Tag 2; 10:10 | 36,22 | 0,98 | 0,13 | 96,60 |
| 5 | Tag 3; 08:10 | 36,13 | 0,99 | 0,13 | 95,91 |
| 6 | Tag 3; 11:20 | 35,57 | 0,97 | 0,12 | 96,49 |
| 7 | Tag 4; 07:05 | 36,30 | 1,05 | 0,22 | 96,83 |
| 8 | Tag 4; 10:00 | 35,98 | 1,09 | 0,23 | 96,22 |
| 9 | Tag 5; 07:40 | 36,42 | 0,90 | 0,21 | 96,83 |
| 10 | Tag 5; 10:05 | 36,26 | 0,90 | 0,19 | 96,32 |
| 11 | Tag 6; 08:10 | 36,21 | 0,94 | 0,18 | 96,37 |
| 12 | Tag 6; 10:55 | 36,10 | 0,93 | 0,20 | 96,39 |
| 13 | Tag 7; 08:40 | 36,35 | 0,87 | 0,23 | 96,79 |
| 14 | Tag 7; 12:45 | 35,98 | 1,09 | 0,20 | 96,42 |
| Mittlere Ausbeute bez. (MASA+MAS) | | | | | Ø 96,4 |
| Schwankungsbreite der Mittleren Ausbeute | | | | | ± 0,46 % |

Beispiel 6 (erfindungsgemäß): Prozess zur Herstellung von Methacrylamid in schwefelsaurer Lösung auf Basis von Acetoncyanhydrin mit strömungs-optimierten Konvertern und optimaler Schwefelsäurekonzentration bei reduzierter Anlagenlast.

Im erfindungsgemäßen Beispiel 6 wurden Rohrumlenkungen wie in Figur 4 (erfindungsgemäß) dargestellt verwendet. Die Ergebnisse, repräsentativ für 7 Tage stationären Betriebes, sind in Tabelle 6 dargestellt. Die Ausbeuteergebnisse der Kombination aus optimaler Schwefelsäurekonzentration und strömungs-optimierten Konvertern bei reduzierter Anlagenlast ist in der nachfolgenden Tabelle 6 abgebildet.

Der erfindungsgemäße Prozess wurde unter den in Tabelle 7 dargestellten Prozessbedingungen durchgeführt. Die genannte Anlagenlast (in %) bezieht sich auf die jeweiligen Zulaufströme (1a + 1b) sowie den im festen Verhältnis hierzu stehenden Schwefelsäure-Strom (2). Abweichend zu den vorangegangenen Beispielen wurde die Anlagenlast in diesem Beispiel von 100%, entsprechend 11000 kg/h Acetoncyanhydrin, auf 75%, entsprechend 8250 kg/h Acetoncyanhydrin, reduziert.

Die mittlere Ausbeute von 14 repräsentativen Proben, gemessen im stationären Betrieb des Prozesses mittels HPLC, betrug unter den genannten Bedingungen 96,8 %.

Weiterhin sind die veränderten Strömungsverhältnisse in den Umlenkungen der Vorwärmer-Segmente (B) bzw. (F) in der CFD-Simulation in Figur 3 dargestellt.

**Tabelle 6: Ergebnisse gemäß Beispiel 6**

| | | Konzentration im Produktstrom [%] | | | Ausbeute [%] |
|---|---|---|---|---|---|
| Probe | Zeit | MASA | MAS | HIBA | MASA + MAS |
| 1 | Tag 1; 08:50 | 35,57 | 0,97 | 0,20 | 96,79 |
| 2 | Tag 1; 12:25 | 36,30 | 1,05 | 0,19 | 96,83 |
| 3 | Tag 2; 09:25 | 35,98 | 1,09 | 0,19 | 96,62 |
| 4 | Tag 2; 10:35 | 36,42 | 0,90 | 0,15 | 96,83 |
| 5 | Tag 3; 14:10 | 36,26 | 0,90 | 0,16 | 96,62 |
| 6 | Tag 3; 16:20 | 36,21 | 0,94 | 0,14 | 96,57 |
| 7 | Tag 4; 07:15 | 36,39 | 0,93 | 0,22 | 97,01 |
| 8 | Tag 4; 10:50 | 36,35 | 0,87 | 0,23 | 96,79 |
| 9 | Tag 5; 07:50 | 36,40 | 0,95 | 0,21 | 97,07 |
| 10 | Tag 5; 10:50 | 36,38 | 0,87 | 0,18 | 96,96 |
| 11 | Tag 6; 07:10 | 35,58 | 0,85 | 0,18 | 96,79 |
| 12 | Tag 6; 10:05 | 36,22 | 0,86 | 0,20 | 96,51 |
| 13 | Tag 7; 08:40 | 36,15 | 0,88 | 0,21 | 96,85 |
| 14 | Tag 7; 11:55 | 36,29 | 0,88 | 0,20 | 96,79 |
| Mittlere Ausbeute bez. (MASA+MAS) | | | | | ∅ 96,8 |
| Schwankungsbreite der Mittleren Ausbeute | | | | | ± 0,28 % |

Tabelle 7 fasst die wichtigsten Prozessparameter sowie Ergebnisse der beschriebenen Beispiele zusammen.

**Tabelle 7: Ausgewählte Prozessparameter der Beispiele 1-6**

| Beispiel | | 1' | 2, | 3 | 4 | 5 | 6 |
|---|---|---|---|---|---|---|---|
| H₂SO₄ -Konzentration | % | 99,7 | 100,6 | 99,7 | 99,5 | 99,5 | 99,5 |
| Acetoncyanhydrin-Qualität | % | 99,0 | 99,0 | 99,0 | 99,0 | 99,0 | 99,0 |
| Massenverhältnis H₂SO₄/ACH | kg/k 9 | 1,62 | 1,62 | 1,62 | 1,62 | 1,62 | 1,62 |
| Anlagenlast | % | 100 | 100 | 100 | 100 | 100 | 75 |
| w_{ø}* in Vorheizer-Segment (B) | m/s | 1,22 | 1,23 | 1,22 | 1,22 | 1,22 | 0,91 |
| w_{ø}* in 90/180° Rohrbögen Vorheizer (B) | m/s | 0,12 | 0,13 | 1,05 | 1,05 | 1,05 | 0,78 |
| w_{ø}* in Verweilzeit-Segment (C) | m/s | 0,23 | 0,24 | 0,23 | 0,23 | 0,23 | 0,17 |
| τ_{ø}** Konvertierung (B + C) | min | 4,82 | 4,80 | 4,62 | 4,62 | 4,62 | 6,16 |
| V_{Linear}/V_{Umlenkung} *** in (B) | | 1,1 | 1,1 | 9,8 | 9,8 | 9,8 | 9,8 |
| V_{Linear}/V_{Umlenkung} *** in (C) | | 28,7 | 28,7 | 28,7 | 28,7 | 28,7 | 28,7 |
| w_{ø}* in Vorheizer-Segment (F) | m/s | 1,48 | 1,49 | 1,48 | 1,48 | 1,48 | 1,13 |
| w_{ø}* in 90/180° Rohrbögen Vorheizer (F) | m/s | 0,14 | 0,15 | 1,28 | 1,28 | 1,28 | 0,97 |
| w_{ø}* in Verweilzeit-Segment (G) | m/s | 0,28 | 0,29 | 0,28 | 0,28 | 0,28 | 0,21 |
| τ_{ø}** Konvertierung (F + G) | min | 4,63 | 4,61 | 4,41 | 4,41 | 4,41 | 5,86 |
| V_{Linear}/V_{Umlankung} *** in (F) | | 1,5 | 1,5 | 13,7 | 13,7 | 13,7 | 13,7 |
| V_{Linear}/V_{Umlenkung} *** in (G) | | 31,0 | 31,0 | 31,0 | 31,0 | 31,0 | 31,0 |
| Mittlere Verweilzeit Pufferbehälter (H) | min | 12 | 12 | 12 | 12 | 51 | 14 |
| Temperatur Pufferbehälter (H) | °C | 100 | 100 | 100 | 100 | 120 | 100 |
| Mittlere Ausbeute (MASA + MAS) bezogen auf ACH | % | ∅ 94,1 | ∅ 90,9 | ∅ 96,5 | ∅ 97,0 | ∅ 96,4 | ∅ 96,8 |
| Streubreite der Ausbeute | % | ± 0.59 | ± 1,87 | ± 0.32 | ± 0.33 | ± 0.46 | ± 0.28 |

| | | | | | | | |
|---|---|---|---|---|---|---|---|
| ') Vergleichsbeispiel *) wØ: Mittlere Strömungsgeschwindigkeit **) τØ: Mittlere Verweilzeit inkl. Rohrleitungen ***) Volumenanteil linearer Reaktorsegmente relativ zum Volumenanteil gebogener Reaktorsegmente | | | | | | | |

Die Ergebnisse in den Tabellen 1-7 verdeutlichen, dass durch die gezielte Reduktion von Rückvermischung in den Vorheizer-Segmenten der jeweiligen Konvertierungsreaktoren ein deutlicher Ausbeutegewinn (Beispiel 3) gegenüber der herkömmlichen Fahrweise (Beispiel 1 bzw. 2) möglich ist. Diese optimalen Ausbeuten sind deutlich weniger Schwankungen unterworfen im Vergleich zu Verfahren, die Parameter und Apparate gemäß Stand der Technik verwenden (vgl. Beispiele 1 und 2).

Die Auswirkung der konstruktiven Veränderungen der Konvertierungsreaktoren auf die Geschwindigkeitsverteilung in den umlenkenden Rohrelementen der Vorheizer-Segmente konnte zudem durch CFD-Berechnungen belegt und visualisiert werden (Figuren 3 und 4).

Weiterhin zeigt sich, dass die Kombination aus der erfindungsgemäß strömungsoptimierten, Rückvermischungs-reduzierten Konvertierung in Verbindung mit einer optimalen Schwefelsäurekonzentration (99,5%) die Erreichung einer maximalen Ausbeute zulässt (Beispiel 4).

Darüber hinaus ist für eine maximale Ausbeute weiterhin entscheidend, dass das erhaltene Reaktionsgemisch gekühlt wird, bevor es in einem Pufferbehälter zwischengelagert wird (Beispiel 4).

Zudem ermöglicht die Kombination aus der erfindungsgemäßen Konverter-Konstruktion und der erfindungsgemäß optimierten Schwefelsäurekonzentration auch bei reduzierter Anlagenlast eine effektive Reduzierung von Rückvermischungs-bedingen Ausbeuteverlusten, so dass auch im Teillastbetrieb hohen Ausbeuten gewährleistet werden können. Insbesondere ermöglicht das erfindungsgemäße Verfahren eine bessere Kontrolle des Verweilzeitprofils der Reaktion auch bei wechselnder Raum-Zeit-Ausbeute innerhalb des verwendeten Reaktors.

### Beispiel 7 (CFD-Simulation):

Die Strömungsverhältnisse in einer Rohrumlenkung gemäß Beispiel 1 und 2 sowie den erfindungsgemäßen Beispielen 3-6 wurden durch CFD-Simulation (Computational Fluid Dynamics) dargestellt. Für die CFD-Simulation wurde eine Grundgesamtheit von 12.000 Volumenelementen betrachtet, deren individuelle Verweilzeit im stationären Strömungszustand berechnet wurde. In den Figuren 3 und 4 sind beispielhaft jeweils 2 Simulationsergebnisse für unterschiedliche Anlagenlasten für das Vergleichsbeispiel (Stand der Technik) (Figuren 3 und 5a sowie Beispiel 1 bzw. 2) sowie für den erfindungsgemäßen Prozess (Figuren 4 und 5b sowie Beispiele 3-6).

Die qualitativen Darstellungen der Strömungsvektoren nach dem Vergleichsbeispiel (rechte Bilder in Figuren 3) zeigen eindeutig Bereiche mit starker Rückvermischung (Z_{RV}) in der Rohrumlenkung, welche sich negativ auf die Verweilzeitverteilung des Reaktionsgemischs auswirken.

Deutlicher wird dies, wenn die Summenverteilung der Rohrumlenkung des Vergleichsbeispiels (Figur 5a) bei unterschiedlichen Durchflussgeschwindigkeiten (Last) betrachtet wird. Zum Beispiel verweilen bei 100% Last (siehe Beispiel 1 bzw. 2), 80% der betrachteten Volumenelemente ca. 1,8 Sekunden in einer 180° Umlenkung (siehe durchgezogene Linie in Figur 5a). Bei reduzierter Anlagenlast auf 75% (Durchfluss) steigt dieser Wert deutlich an und beträgt 3,2 Sekunden (siehe gestrichelte Linie in Figur 5a). Zusätzlich ist die mittlere Verweilzeit der restlichen 10% des Reaktionsgemischs > 5 Sekunden. Wird die große Anzahl von Umlenkungen im Vorheizer-Segment berücksichtig, ergibt sich für min. 10% des Reaktionsgemischs eine deutlich längere Verweilzeit im Reaktor, was letztendlich zu den gemessenen Ausbeuteeinbrüchen führt.

Die Ergebnisse der Strömungssimulation in einer Rohrumlenkung gemäß erfindungsgemäßem Beispiel (Figuren 4 und 5b) zeigen im Vergleich zum Vergleichsbeispiel, dass die Bereiche mit starker Rückvermischung, welche sich negativ auf die Verweilzeitverteilung des Reaktionsgemischs auswirken, eliminiert werden konnten. Verdeutlicht wird dies, neben der qualitativen Darstellung der Strömungsvektoren (rechte Bilder in Figur 4), wenn die Summenverteilung bei unterschiedlichen Durchflussgeschwindigkeiten betrachtet wird (Figur 5b). Im gezeigten Beispiel verweilen bei 100% Last (vgl. Beispiel 3), 80% der betrachteten Volumenelemente nur 0,55 Sekunden, bei Teillast (75%) nur 1,0 Sekunden in einer 180° Umlenkung. Weiterhin ist die deutlich schmalere Verweilzeitverteilung dieser Konstruktion zu erkennen, da die Volumenelemente mit einer Verweilzeit > 1 Sekunden nach spätestens 3 Sekunden aus der Umlenkung austreten, was im Vergleichsbeispiel 1 in Figur 5a zum Beispiel nicht der Fall ist.

## Patentansprüche

1. Verfahren zur Herstellung von Methylmethacrylat und/oder Methacrylsäure, umfassend die Verfahrensschritte:
a. die Umsetzung von Acetoncyanhydrin und Schwefelsäure in ein oder mehreren Reaktoren I in einer ersten Reaktionsstufe bei einer Temperatur im Bereich von 70°C bis 130°C, wobei eine erste Reaktionsmischung, enthaltend Sulfoxyisobuttersäureamid und Methacrylsäureamid, erhalten wird;
b. das Konvertieren der ersten Reaktionsmischung, umfassend das Erhitzen auf eine Temperatur im Bereich von 130 bis 200 °C, in ein oder mehreren Reaktoren II in einer zweiten Reaktionsstufe, wobei eine zweite Reaktionsmischung, enthaltend überwiegend Methacrylsäureamid und Schwefelsäure, erhalten wird; und
c. die Umsetzung der zweiten Reaktionsmischung mit Wasser und optional Methanol, in ein oder mehreren Reaktoren III in einer dritten Reaktionsstufe, wobei eine dritte Reaktionsmischung, enthaltend Methacrylsäure und/oder Methylmethacrylat, erhalten wird;
wobei
(i) die in der ersten Reaktionsstufe eingesetzte Schwefelsäure, welche an einer oder mehreren Stellen der Reaktoren I zugeführt wird, eine Konzentration im Bereich von 98,0 Gew.-% bis 100,5 Gew.-% aufweist,
(ii) die Verweilzeit der ersten Reaktionsmischung in der zweiten Reaktionsstufe im Bereich von 2 bis 15 min liegt,
(iii) das Erhitzen in der zweiten Reaktionsstufe in einem oder mehreren Reaktoren II durchgeführt wird, wobei mindestens ein Reaktor II mindestens ein Vorheizer-Segment umfasst, welches ein oder mehrere lineare Rohrleitungselemente umfasst, die mittels eines räumlich von der Reaktionsmischung getrennten Heizmediums aufgeheizt werden, wobei die Reaktionsmischung um 10 bis 100°C erhitzt wird,
(iv) das Konvertieren in der zweiten Reaktionsstufe in einem oder mehreren Reaktoren II durchgeführt wird, wobei mindestens ein Reaktor II mindestens ein Verweilzeit-Segment umfasst, welches annähernd adiabatisch betrieben wird,
(v) das Vorheizer-Segment und/oder das Verweilzeit-Segment in einer Kombination aus linearen Rohrleitungselementen und Umlenkungen realisiert sind, wobei die linearen Rohrleitungselementen und die Umlenkungen über Reduzierflansche miteinander verbunden sind,
(vi) die in Schritt b erhaltene zweite Reaktionsmischung, enthaltend überwiegend Methacrylsäureamid und Schwefelsäure, optional auf eine Temperatur unterhalb von 120 °C abgekühlt wird, und/oder optional in einem Zwischenbehälter zwischengepuffert wird, bevor die Reaktionsmischung in die dritte Reaktionsstufe geführt wird.
wobei das Leervolumen der linearen Rohrleitungselemente relativ zum Leervolumen der Umlenkungen in mindestens einem Vorheizer-Segment einem Verhältnis im Bereich von 2,0 bis 75,0 entspricht.

2. Verfahren nach Anspruch 1, **dadurch gekennzeichnet, dass** im Vorheizer-Segment die linearen Rohrleitungselemente in ein oder mehreren geraden Rohrleitungen ausgeführt sind, die einen inneren Durchmesser im Bereich von 8 mm bis 200 mm aufweisen, wobei die Rohrleitungen der linearen Rohrleitungselemente im Vorheizer-Segment einen kleineren inneren Durchmesser aufweisen als die Rohrleitungen der linearen Rohrleitungselemente im Verweilzeit-Segment.

3. Verfahren nach mindestens einem der Ansprüchen 1 bis 2, **dadurch gekennzeichnet, dass** die linearen Rohrleitungselemente im Vorheizer-Segment und/oder im Verweilzeit-Segment des mindestens einen Reaktors II in parallelen Rohrbündeln ausgeführt sind und diese Rohrbündel mittels mindestens einer Umlenkung verbunden sind, wobei die Umlenkung als Rohrleitung ausgestaltet ist, welches einen ersten Abschnitt, der einen in Strömungsrichtung konvergent verlaufenden inneren Querschnitt aufweist, einen zweiten Abschnitt, der eine im Wesentlichen konstante innere Querschnittsfläche aufweist, und einen dritten Abschnitt, der einen in Strömungsrichtung divergent verlaufenden inneren Querschnitt aufweist, umfasst.

4. Verfahren nach mindestens einem der Ansprüchen 1 bis 3, **dadurch gekennzeichnet, dass** mindestens ein Vorheizer-Segment lineare Rohrleitungselemente und Umlenkungen umfasst, wobei die linearen Rohrleitungselemente des Vorheizer-Segments 1 bis 50 separate gerade Rohrleitungen umfassen, die mit einem Heizmedium sekundär beheizt werden und parallel zueinander angeordnet sind, und wobei die Umlenkungen, welche die linearen Rohrleitungselemente miteinander verbinden, mindestens eine Rohrleitung umfassen.

5. Verfahren nach mindestens einem der Ansprüche 1 bis 4, **dadurch gekennzeichnet, dass** die Umlenkungen aus Rohrleitungselementen gebildet sind, die eine Biegung von 90° bis 180°aufweisen.

6. Verfahren nach mindestens einem der Ansprüche 1 bis 5, **dadurch gekennzeichnet, dass** innerhalb der Umlenkungen eine mittlere Geschwindigkeit der Reaktionsmischung von 0,2 bis 3 m/sec eingestellt wird und die Abweichung der lokalen Geschwindigkeit an jeder Stelle innerhalb der Umlenkungen von der mittleren Fördergeschwindigkeit kleiner oder gleich 30% beträgt.

7. Verfahren nach mindestens einem der Ansprüche 1 bis 6, **dadurch gekennzeichnet, dass** bei der Durchführung der ersten Reaktionsstufe und der zweiten Reaktionsstufe mindestens eine Zwischenkonvertierung zwischen mindestens zwei ersten Reaktionsstufe stattfindet, wobei die mindestens zwei Reaktoren II der Konvertierung durch mindestens einen Rohrbündelwärmetauscher als Vorheizer-Segment, verbunden mit mindestens einer Verweilzeitstrecke als Verweilzeit-Segment, realisiert sind.

8. Verfahren nach Anspruch 7, **dadurch gekennzeichnet, dass** mindestens zwei Reaktoren I verwendet werden, wobei mindestens ein Reaktor II umfassend mindestens ein Vorheizer- und/oder Verweilzeit-Segment zwischen den Reaktoren I sowie mindestens ein Reaktor II umfassend mindestens ein Vorheizer- und/oder Verweilzeit-Segment am Austritt der ersten Reaktionsstufe, zum Einsatz kommen.

9. Verfahren nach mindestens einem der Ansprüche 1 bis 8, **dadurch gekennzeichnet, dass** mindestens 60% der statischen Verweilzeit von mindestens einer zweiten Reaktionsstufe in einem als Rohrleitung ausgeführten Verweilzeit-Segment realisiert sind.

10. Verfahren nach mindestens einem der Ansprüche 1 bis 9, **dadurch gekennzeichnet, dass** die erste Reaktionsmischung resultierend aus der ersten Reaktionsstufe in einem konstant-geregeltem Massenstrom mittels einer Austragspumpe, ausgehend von mindestens einem Reaktor I durch mindestens einen Reaktor II gefördert wird.

11. Verfahren nach mindestens einem der Ansprüche 1 bis 10, **dadurch gekennzeichnet, dass** in mindestens einem Reaktor II mindestens ein Vorheizer-Segment als Rohrbündelwärmetauscher ausgestaltet ist, wobei der Rohrbündelwärmetauscher rohrseitig mit der Reaktionsmischung durchströmt wird und durch die freie Querschnittsfläche der verwendeten Rohre des Rohrbündelwärmetauschers eine mittlere Strömungsgeschwindigkeit von mindestens 0,2 m/s in den Rohren gewährleistet ist.

12. Apparat zur Durchführung des Verfahrens nach einem der Ansprüche 1 bis 11, wobei der Apparat jeweils einen oder mehrere Reaktoren I, II und III aufweist, welche miteinander durch Rohrleitungselemente verbunden sind, und wobei mindestens ein Reaktor II mindestens ein Vorheizer-Segment und mindestens ein Verweilzeit-Segment umfasst, wobei das Vorheizer-Segment ein oder mehreren lineare Rohrleitungselemente umfasst, die mittels eines räumlich von der Reaktionsmischung getrennten Heizmedium aufgeheizt werden, und wobei das Verweilzeit-Segment annähernd adiabatisch betrieben wird,
wobei das Vorheizer-Segment und/oder das Verweilzeit-Segment in einer Kombination aus linearen Rohrleitungselementen und Umlenkungen realisiert sind,
wobei die linearen Rohrleitungselementen und die Umlenkungen über Reduzierflansche miteinander verbunden sind, und wobei
das Leervolumen der linearen Rohrleitungselemente relativ zum Leervolumen der Umlenkungen in mindestens einem Vorheizer-Segment einem Verhältnis im Bereich von 2,0 bis 75,0 entspricht.

13. Apparat nach Anspruch 12, **dadurch gekennzeichnet, dass** im Vorheizer-Segment die linearen Rohrleitungselemente in ein oder mehreren geraden Rohrleitungen ausgeführt werden, die einen inneren Durchmesser im Bereich von 8 mm bis 200 mm aufweisen, wobei die Rohrleitungen der linearen Rohrleitungselemente im Vorheizer-Segment einen kleineren inneren Durchmesser aufweisen als die Rohrleitungen der linearen Rohrleitungselemente im Verweilzeit-Segment.

14. Apparat nach mindestens einem der Ansprüche 12 oder 13, **dadurch gekennzeichnet, dass** die Umlenkungen aus Rohrleitungselementen gebildet werden, die eine Biegung von 90° bis 180°aufweisen.

15. Apparat nach mindestens einem der Ansprüche 12 bis 14, **dadurch gekennzeichnet, dass** die linearen Rohrleitungselemente des Vorheizer-Segments und/oder des Verweilzeit-Segments durch Umlenkungen verbunden sind, wobei die Umlenkungen ein um 180° umlenkendes Element darstellen, das gebildet wird aus zwei 90 °-Rohrleitungsumlenkungen und einem linearen Rohrleitungselement, das bevorzugt zwischen den 90 °-Rohrleitungsumlenkungen angeordnet ist.

## Claims

1. Process for preparing methyl methacrylate and/or methacrylic acid, comprising the process steps of:
a. the reacting of acetone cyanohydrin and sulfuric acid in one or more reactors I in a first reaction stage at a temperature in the range from 70°C to 130°C to obtain a first reaction mixture comprising sulfoxyisobutyramide and methacrylamide;
b. the converting of the first reaction mixture, comprising heating to a temperature in the range from 130 to 200°C, in one or more reactors II in a second reaction stage to obtain a second reaction mixture comprising predominantly methacrylamide and sulfuric acid; and
c. the reacting of the second reaction mixture with water and optionally methanol in one or more reactors III in a third reaction stage to obtain a third reaction mixture comprising methacrylic acid and/or methyl methacrylate;
wherein
(i) the sulfuric acid used in the first reaction stage, which is fed in at one or more points in the reactors I, has a concentration in the range from 98.0% by weight to 100.5% by weight,
(ii) the dwell time of the first reaction mixture in the second reaction stage is in the range from 2 to 15 min,
(iii)the heating in the second reaction stage is performed in one or more reactors II, wherein at least one reactor II comprises at least one preheater segment comprising one or more linear pipeline elements that are heated by means of a heating medium spatially separate from the reaction mixture, wherein the reaction mixture is heated by 10 to 100°C,
(iv) the converting in the second reaction stage is performed in one or more reactors II, wherein at least one reactor II comprises at least one delay segment which is operated under virtually adiabatic conditions,
(v) the preheater segment and/or the delay segment are implemented in a combination of linear pipeline elements and deflections, wherein the linear pipeline elements and the deflections are connected to one another via reducing flanges,
(vi) the second reaction mixture obtained in step b, comprising predominantly methacrylamide and sulfuric acid, is optionally cooled down to a temperature below 120°C, and/or optionally intermediately buffered in an intermediate vessel, before the reaction mixture is guided into the third reaction stage,
wherein the superficial volume of the linear pipeline elements relative to the superficial volume of the deflections in at least one preheater segment corresponds to a ratio in the range from 2.0 to 75.0.

2. Process according to Claim 1, **characterized in that**, in the preheater segment, the linear pipeline elements are executed in one or more straight pipelines having an internal diameter in the range from 8 mm to 200 mm, wherein the pipelines of the linear pipeline elements in the preheater segment have a smaller internal diameter than the pipelines of the linear pipeline elements in the delay segment.

3. Process according to at least one of Claims 1 to 2, **characterized in that** the linear pipeline elements in the preheater segment and/or in the delay segment of the at least one reactor II are executed in parallel bundles of tubes, and these bundles of tubes are connected by means of at least one deflection, wherein the deflection is configured as a pipeline comprising a first section having an internal cross section that runs convergently in flow direction, a second section having an essentially constant internal cross-sectional area, and a third section having an internal cross section that runs divergently in flow direction.

4. Process according to at least one of Claims 1 to 3, **characterized in that** at least one preheater segment comprises linear pipeline elements and deflections, wherein the linear pipeline elements of the preheater segment comprise 1 to 50 separate straight pipelines that are secondarily heated with a heating medium and are arranged parallel to one another, and wherein the deflections that connect the linear pipeline elements to one another comprise at least one pipeline.

5. Process according to at least one of Claims 1 to 4, **characterized in that** the deflections are formed from pipeline elements having an inflection of 90° to 180°.

6. Process according to at least one of Claims 1 to 5, **characterized in that** an average speed of the reaction mixture of 0.2 to 3 m/sec is established within the deflections and the variance in the local speed at any point within the deflections from the average conveying speed is not more than 30%.

7. Process according to at least one of Claims 1 to 6, **characterized in that** at least one intermediate conversion between at least two first reaction stages takes place in the performance of the first reaction stage and the second reaction stage, wherein the at least two reactors II for the conversion are implemented by at least one shell-and-tube heat exchanger as preheater segment, combined with at least one delay zone as delay segment.

8. Process according to Claim 7, **characterized in that** at least two reactors I are used, wherein at least one reactor II comprising at least one preheater segment and/or delay segment between the reactors I, and at least one reactor II comprising at least one preheater segment and/or delay segment at the exit from the first reaction stage are used.

9. Process according to at least one of Claims 1 to 8, **characterized in that** at least 60% of the static dwell time of at least one second reaction stage is implemented in a delay segment executed as a pipeline.

10. Process according to at least one of Claims 1 to 9, **characterized in that** the first reaction mixture resulting from the first reaction stage, proceeding from at least one reactor I, is conveyed through at least one reactor II at a constantly controlled mass flow rate by means of a discharge pump.

11. Process according to at least one of Claims 1 to 10, **characterized in that** at least one preheater segment in at least one reactor II is configured as a shell-and-tube heat exchanger, wherein the reaction mixture flows through the shell-and-tube heat exchanger on the tube side, and the free cross-sectional area of the tubes used in the shell-and-tube heat exchanger ensures an average flow rate of at least 0.2 m/s in the tubes.

12. Apparatus for performance of the process according to any of Claims 1 to 11, wherein the apparatus in each case comprises one or more reactors I, II and III that are connected to one another by pipeline elements, and wherein at least one reactor II comprises at least one preheater segment and at least one delay segment, wherein the preheater segment comprises one or more linear pipeline elements that are heated by means of a heating medium spatially separate from the reaction mixture, and wherein the delay segment is operated under virtually adiabatic conditions,
wherein the preheater segment and/or the delay segment are implemented in a combination of linear pipeline elements and deflections, wherein the linear pipeline elements and the deflections are connected to one another via reducing flanges, and wherein
the superficial volume of the linear pipeline elements relative to the superficial volume of the deflections in at least one preheater segment corresponds to a ratio in the range from 2.0 to 75.0.

13. Apparatus according to Claim 12, **characterized in that**, in the preheater segment, the linear pipeline elements are executed in one or more straight pipelines having an internal diameter in the range from 8 mm to 200 mm, wherein the pipelines of the linear pipeline elements in the preheater segment have a smaller internal diameter than the pipelines of the linear pipeline elements in the delay segment.

14. Apparatus according to at least one of Claims 12 or 13, **characterized in that** the deflections are formed from pipeline elements having an inflection of 90° to 180°.

15. Apparatus according to at least one of Claims 12 to 14, **characterized in that** the linear pipeline elements of the preheater segment and/or of the delay segment are connected by deflections, wherein the deflections are an element that causes deflection by 180°, formed from two 90° pipeline deflections and one linear pipeline element preferably disposed between the 90° pipeline deflections.

## Revendications

1. Procédé de préparation de méthacrylate de méthyle et/ou d'acide méthacrylique, comprenant les étapes de procédé :
a. la transformation de cyanhydrine d'acétone et d'acide sulfurique dans un ou plusieurs réacteurs I dans un premier stade de réaction à une température dans la plage de 70 °C à 130 °C, un premier mélange réactionnel, contenant de l'amide de l'acide sulfoxyisobutyrique et du méthacrylamide, étant obtenu ;
b. la conversion du premier mélange réactionnel, comprenant le chauffage à une température dans la plage de 130 à 200 °C, dans un ou plusieurs réacteurs II dans un deuxième stade de réaction, un deuxième mélange réactionnel, contenant principalement du méthacrylamide et de l'acide sulfurique, étant obtenu ; et
c. la transformation du deuxième mélange réactionnel avec de l'eau et éventuellement du méthanol, dans un ou plusieurs réacteurs III dans un troisième stade de réaction, un troisième mélange réactionnel, contenant de l'acide méthacrylique et/ou du méthacrylate de méthyle, étant obtenu ;
(i) l'acide sulfurique utilisé dans le premier stade de réaction, qui est alimenté au niveau d'un ou plusieurs emplacements des réacteurs I, présentant une concentration dans la plage de 98,0 % en poids à 100,5 % en poids,
(ii) la durée de séjour du premier mélange réactionnel dans le deuxième stade de réaction se situant dans la plage de 2 à 15 min,
(iii) le chauffage dans le deuxième stade de réaction étant réalisé dans un ou plusieurs réacteurs II, au moins un réacteur II comprenant au moins un segment de préchauffeur, qui comprend un ou plusieurs éléments de canalisation linéaires, qui sont chauffés au moyen d'un moyen de chauffage à distance du mélange réactionnel, le mélange réactionnel étant chauffé de 10 à 100 °C,
(iv) la conversion dans le deuxième stade de réaction étant réalisée dans un ou plusieurs réacteurs II, au moins un réacteur II comprenant au moins un segment de durée de séjour qui est exploité de manière presque adiabatique,
(v) le segment de préchauffeur et/ou le segment de durée de séjour étant réalisés en une combinaison d'éléments de canalisation linéaires et de déviations, les éléments de canalisation linéaires et les déviations étant reliés ensemble par l'intermédiaire de brides de réduction,
(vi) le deuxième mélange réactionnel obtenu dans l'étape b, contenant principalement du méthacrylamide et de l'acide sulfurique, étant éventuellement refroidi à une température en dessous de 120 °C et/ou éventuellement placé dans un récipient intermédiaire, avant que le mélange réactionnel ne soit conduit dans le troisième stade de réaction,
le volume libre des éléments de canalisation linéaires par rapport au volume libre des déviations dans au moins un segment de préchauffeur correspondant à un rapport dans la plage de 2,0 à 75,0.

2. Procédé selon la revendication 1, **caractérisé en ce que** dans le segment de préchauffeur les éléments de canalisation linéaires sont réalisés en une ou plusieurs canalisations droites qui présentent un diamètre interne dans la plage de 8 mm à 200 mm, les canalisations des éléments de canalisation linéaires dans le segment de préchauffeur présentant un diamètre interne plus petit que les canalisations des éléments de canalisation linéaires dans le segment de durée de séjour.

3. Procédé selon au moins l'une quelconque des revendications 1 et 2, **caractérisé en ce que** les éléments de canalisation linéaires dans le segment de préchauffeur et/ou dans le segment de durée de séjour de l'au moins un réacteur II sont réalisés en faisceaux tubulaires parallèles et ces faisceaux tubulaires sont reliés au moyen d'au moins une déviation, la déviation étant conçue comme canalisation qui comprend une première partie qui présente une section transversale interne s'étendant de manière convergente dans la direction d'écoulement, une deuxième partie qui présente une surface de section transversale interne essentiellement constante, et une troisième partie qui présente une section transversale interne s'étendant de manière divergente dans la direction d'écoulement.

4. Procédé selon au moins l'une quelconque des revendications 1 à 3, **caractérisé en ce qu'**au moins un segment de préchauffeur comprend des éléments de canalisation linéaires et des déviations, les éléments de canalisation linéaires du segment de préchauffeur comprenant 1 à 50 canalisations droites distinctes qui sont chauffées de manière secondaire par un moyen de chauffage et sont agencées de manière parallèle les unes par rapport aux autres, et les déviations qui relient ensemble les éléments de canalisation linéaires comprenant au moins une canalisation.

5. Procédé selon au moins l'une quelconque des revendications 1 à 4, **caractérisé en ce que** les déviations sont formées à partir d'éléments de canalisation qui présentent une courbure de 90° à 180°.

6. Procédé selon au moins l'une quelconque des revendications 1 à 5, **caractérisé en ce qu'**à l'intérieur des déviations, une vitesse moyenne du mélange réactionnel est ajustée de 0,2 à 3 m/s et la variation de la vitesse locale en chaque endroit à l'intérieur des déviations est égale ou inférieure à 30 % de la vitesse de transport moyenne.

7. Procédé selon au moins l'une quelconque des revendications 1 à 6, **caractérisé en ce que** lors de la mise en œuvre du premier stade de réaction et du deuxième stade de réaction, au moins une conversion intermédiaire entre au moins deux premiers stades de réaction a lieu, les au moins deux réacteurs II de la conversion étant réalisés par au moins un échangeur de chaleur à faisceaux tubulaires en tant que segment de préchauffeur, relié avec au moins un trajet de durée de séjour en tant que segment de durée de séjour.

8. Procédé selon la revendication 7, **caractérisé en ce qu'**au moins deux réacteurs I sont utilisés, au moins un réacteur II comprenant au moins un segment de préchauffeur et/ou de durée de séjour entre les réacteurs I ainsi qu'au moins un réacteur II comprenant au moins un segment de préchauffeur et/ou de durée de séjour à la sortie du premier stade de réaction, étant utilisés.

9. Procédé selon au moins l'une quelconque des revendications 1 à 8, **caractérisé en ce qu'**au moins 60 % de la durée de séjour statique d'au moins un deuxième stade de réaction sont réalisés dans un segment de durée de séjour réalisé comme canalisation.

10. Procédé selon au moins l'une quelconque des revendications 1 à 9, **caractérisé en ce que** le premier mélange réactionnel résultant du premier stade de réaction est transporté dans un flux de masse régulé de manière constante au moyen d'une pompe de décharge, en provenance d'au moins un réacteur I par au moins un réacteur II.

11. Procédé selon au moins l'une quelconque des revendications 1 à 10, **caractérisé en ce que** dans au moins un réacteur II au moins un segment de préchauffeur est agencé comme échangeur de chaleur à faisceaux tubulaires, l'échangeur de chaleur à faisceaux tubulaires étant traversé du côté tube par le mélange réactionnel et, par la surface de section transversale libre des tubes utilisés de l'échangeur de chaleur à faisceaux tubulaires, une vitesse d'écoulement moyenne d'au moins 0,2 m/s étant assurée dans les tubes.

12. Appareil pour la mise en œuvre du procédé selon l'une quelconque des revendications 1 à 11, l'appareil présentant à chaque fois un ou plusieurs réacteurs I, II et III, qui sont reliés les uns aux autres par des éléments de canalisation, et au moins un réacteur II comprenant au moins un segment de préchauffeur et au moins un segment de durée de séjour, le segment de préchauffeur comprenant un ou plusieurs éléments de canalisation linéaires, qui sont chauffés au moyen d'un moyen de chauffage à distance du mélange réactionnel, et le segment de durée de séjour étant exploité de manière presque adiabatique, le segment de préchauffeur et/ou le segment de durée de séjour étant réalisés dans une combinaison d'éléments de canalisation linéaires et de déviations, les éléments de canalisation linéaires et les déviations étant reliés ensemble par l'intermédiaire de brides de réduction,
le volume libre des éléments de canalisation linéaires par rapport au volume libre des déviations dans au moins un segment de préchauffeur correspondant à un rapport dans la plage de 2,0 à 75,0.

13. Appareil selon la revendication 12, **caractérisé en ce que** dans le segment de préchauffeur les éléments de canalisation linéaires sont réalisés en une ou plusieurs canalisations droites qui présentent un diamètre interne dans la plage de 8 mm à 200 mm, les canalisations des éléments de canalisation linéaires dans le segment de préchauffeur présentant un diamètre interne plus petit que les canalisations des éléments de canalisation linéaires dans le segment de durée de séjour.

14. Appareil selon au moins l'une quelconque des revendications 12 ou 13, **caractérisé en ce que** les déviations sont formées à partir d'éléments de canalisation qui présentent une courbure de 90° à 180°.

15. Appareil selon au moins l'une quelconque des revendications 12 à 14, **caractérisé en ce que** les éléments de canalisation linéaires du segment de préchauffeur et/ou du segment de durée de séjour sont reliés par des déviations, les déviations représentant un élément dévié de 180° qui est formé à partir de deux déviations de canalisation à 90° et d'un élément de canalisation linéaire qui est préférablement agencé entre les déviations de canalisation à 90°.
